# EUROPEAN PATENT SPECIFICATION

(11) **EP 2 772 534 B1**
(45) Date of publication and mention of the grant of the patent: **18.09.2019**
(21) Application number: 12844458.5
(22) Date of filing: 26.10.2012
(51) Int. Cl.: C12N 5/071, A61K 35/38, A61P 1/00, A61P 1/04, C12N 5/074

(54) **CULTURING COLORECTAL EPITHELIAL STEM CELLS AND TRANSPLANTING COLORECTAL EPITHELIUM**
KULTIVIERUNG KOLOREKTALER EPITHELSTAMMZELLEN UND TRANSPLANTATION EINES KOLOREKTALEN EPITHELS
CULTURE DE CELLULE SOUCHE ÉPITHÉLIALE COLORECTALE, ET TRANSPLANTATION D'ÉPITHÉLIUM COLORECTAL

(30) Priority: 27.10.2011 JP 2011236469
(43) Date of publication of application: 03.09.2014
(73) Proprietor: National University Corporation Tokyo Medical and Dental University, Bunkyo-ku Tokyo 113-8510 (JP)
(72) Inventor: WATANABE, Mamoru, Tokyo 113-8510 (JP); NAKAMURA, Tetsuya, Tokyo 113-8510 (JP)
(74) Representative: Mewburn Ellis LLP
(86) International application number: PCT/JP2012/006897
(87) International publication number: WO 2013/061608

(56) References cited:
- WO-A2-2010/090513
- WO-A2-2011/159359
- WO-A2-2012/168930
- US-A1- 2012 196 312
- WHITEHEAD ET AL: "Clonogenic growth of epithelial cells from normal colonic mucosa from both mice and humans", GASTROENTEROLOGY, ELSEVIER, PHILADELPHIA, PA, vol. 117, no. 4, October 1999 (1999-10), pages 858-865, XP005145666, ISSN: 0016-5085, DOI: 10.1016/S0016-5085(99)70344-6
- YUI S. ET AL.: 'Functional engraftment of colon epithelium expanded in vitro from a single adult Lgr5+ stem cell' NATURE MEDICINE vol. 18, no. 4, 11 March 2012, pages 618 - 623, XP003031622
- JUNG P. ET AL.: 'Isolation and in vitro expansion of human colonic stem cells' NATURE MEDICINE vol. 17, no. 10, 04 September 2011, pages 1225 - 1227, XP055069087
- SATO T. ET AL.: 'Long-term Expansion of Epithelial Organoids From Human Colon, Adenoma, Adenocarcinoma, and Barrett's Epithelium' GASTROENTEROLOGY vol. 141, 05 September 2011, pages 1762 - 1772, XP028325676
- WEIGMANN B. ET AL.: 'Isolation and subsequent analysis of murine lamina propria mononuclear cells from colonic tissue' NATURE PROTOCOLS vol. 2, no. 10, 2007, pages 2307 - 2311, XP055149958
- LIU S. ET AL.: 'Isolation and characterization of human spermatogonial stem cells' REPRODUCTIVE BIOLOGY AND ENDOCRINOLOGY vol. 9, no. 141, 24 October 2011, pages 1 - 9, XP021112126
- CHE X. ET AL.: 'Rapid isolation of muscle-derived stem cells by discontinuous Percoll density gradient centrifugation' IN VITRO CELLULAR & DEVELOPMENTAL BIOLOGY - ANIMAL vol. 47, 21 June 2011, pages 454 - 458, XP019930512
- SATO T. ET AL.: 'Single Lgr5 stem cells build crypt-villus structures in vitro without a mesenchymal niche' NATURE vol. 459, 14 May 2009, pages 262 - 265, XP002529277
- TOMOHIRO MIZUTANI ET AL.: 'Recent advancement in intestinal epithelial stem cell research' THE CELL, 2011 NEN 7 GATSU vol. 43, no. 8, pages 286 - 289, XP008174232

## Description

### Technical Field

The present invention relates to (A) a colorectal epithelial stem cell and/or a colorectal epithelial cell and (B) an extracellular matrix for use in the prevention or treatment of a human bowel disease.

### Background Art

There is increasing interest in a potential for application of adult stem cells to therapy of gastrointestinal diseases. Gastrointestinal epithelial stem cells, having self-renewal ability and generating ability, of epithelial cells of stomach, small intestine, and colon play important roles for maintaining tissue homeostasis at each site of gastrointestinal tracts (Non-patent Documents 1 to 3). Findings for biology of gastrointestinal stem cells have notably increased by recent studies. It has been suggested that some signaling pathways including Wnt, BMP, and Notch cascades act together with each other to regulate the maintenance of stem cells and to control the homeostasis of the epitheliums (Non-patent Document 4). A Wnt target gene, Lgr5, was identified as a marker of stem cells at the bases of the crypts of the small intestine and the colon (Non-patent Document 5) and the base of a gastric unit (Non-patent Document 6) by studies using lineage tracking.

At present, gastrointestinal stem cells can be recognized with a certain marker, and cell-based therapy using gastrointestinal stem cells has been being expected to fundamentally change the therapy of many refractory diseases. However, to this day, there is no report on the use of gastrointestinal epithelial cells isolated and propagated by culturing in vitro as a material for cell-based therapy to repair a damaged tissue. In addition, it has not been yet revealed whether cultured and propagated gastrointestinal epithelial stem cells can repair the tissue at the damaged site. It is quite recently that long-term culture of intestinal small fragments including epithelial and mesenchymal components has become possible (Non-patent Document 7).

Non-patent Document 8 describes a method of culturing colorectal epithelial stem cells by embedding isolated colonic crypts in matrigel and adding a complete crypt culture medium thereto. The complete crypt culture medium is composed of Advanced DMEM/F12 as a basal medium, a Wnt3a or Wnt3a conditioned medium, EGF, Noggin, r-spondin-1, B-27 (registered trademark) supplement (manufactured by Invitrogen), gastrin, nicotinamide, A83-01 (TGF-β1 receptor kinase inhibitor), SB202190 (p38 inhibitor), etc. The exact composition of B-27 (registered trademark) supplement is not disclosed, but it is known that the supplement contains biotin, L-carnitine, corticosterone, ethanolamine, D(+)-galactose, reduced glutathione, linoleic acid, linolenic acid, progesterone, putrescine, retinyl acetate, selenium, triiodo-1-thyronine, vitamin E, vitamin E acetate, bovine albumin, catalase, insulin, superoxide dismutase, transferrin, and so on (Non-patent Document 9). In the case of culturing colorectal epithelial stem cells in vitro in a serum-free culture medium, gastrin, nicotinamide, A83-01 (TGF-β1 receptor kinase inhibitor), SB202190 (p38 inhibitor), and B-27 supplement are thought to be essential components. Among them, it has been believed that B-27 supplement is particularly indispensable.

Meanwhile, bovine serum albumin (BSA) is albumin contained in bovine serum in a large amount and is known to function as a carrier for fatty acid and trace elements. Wnt3a is a protein encoded by a WNT3A gene and is known to activate the β-catenin pathway. The Wnt/β-catenin pathway is known to be involved in proliferation of stem cells and maintenance of undifferentiation. R-spondin-1 (Roof-plate-specific Spondin-1: Rspo1) is a protein encoded by an RSPO1 gene and is known to contribute to differentiation of the dorsal neural tube and to enhance the proliferation of intestinal crypt epithelial cells. An epidermal growth factor (EGF) is a protein encoded by an EGF gene and is known to enhance the proliferation of epithelial cells. A hepatocyte growth factor (HGF) is a protein encoded by an HGF gene and is known to enhance the proliferation of various cells including hepatocytes. Noggin is a protein encoded by an NOG gene and is known to inhibit the signaling of transforming growth factor beta (TGF-β).

However, it has not been known that colorectal epithelial stem cells or the like isolated from colon tissue of a mammal such as a mouse or a human being can be maintained or propagated for a long time in vitro even in a serum-free culture medium by using a culture medium containing serum albumin, Wnt3a, and r-spondin-1 in addition to the basal medium components (a carbon source such as a saccharide, a nitrogen source such as an amino acid, and an inorganic salt).

Patent Document 1 describes a method for culturing epithelial stem cells and culturing the cells in the presence of a Bone Morphogenetic Protein (BMP) inhibitor, a mitogenic growth factor, and a Wnt agonist. Patent Document 1 also describes a cell culture medium comprising a BMP inhibitor, a mitogenic growth factor, and a Wnt agonist.

### Prior Art Documents

### Patent Documents

Patent Document 1: WO 2010/090513 A2

### Non-patent Documents

Non-patent Document 1: Potten, C.S., Booth, C. & Pritchard, D.M., The intestinal epithelial stem cell: the mucosal governor, Int. J. Exp. Pathol., 78, 219-243 (1997).
Non-patent Document 2: Bjerknes, M. & Cheng, H., Intestinal epithelial stem cells and progenitors, Methods Enzymol., 419, 337-383 (2006).
Non-patent Document 3: Barker, N., van de Wetering, M. & Clevers, H., The intestinal stem cell, Genes Dev., 22, 1856-1864 (2008).
Non-patent Document 4: Crosnier, C., Stamataki, D. & Lewis, J., Organizing cell renewal in the intestine: stem cells, signals and combinatorial control, Nat. Rev. Genet., 7, 349-359 (2006).
Non-patent Document 5: Barker, N., et al., Identification of stem cells in small intestine and colon by marker gene Lgr5, Nature, 449, 1003-1007 (2007).
Non-patent Document 6: Barker, N., et al., Lgr5 (+ve) stem cells drive self-renewal in the stomach and build long-lived gastric units in vitro, Cell Stem Cell, 6, 25-36 (2010).
Non-patent Document 7: Sato, T., et al., Single Lgr5 stem cells build crypt-villus structures in vitro without a mesenchymal niche, Nature, 459, 262-265 (2009).
Non-patent Document 8: Sato, T., et al., Long-term Expansion of Epithelial Organoids From Human Colon, Adenoma, Adenocarcinoma, and Barrett's Epithelium, Gastroenterology, DOI: 10.1053/j.gastro.2011.07.050 (2011).
Non-patent Document 9: G.J. Brewer, et al., Optimized survival of hippocampal neurons in B27-supplemented Neurobasal, a new serum-free medium combination, J. Neurosci. Res., 35, 567-576 (1993).

### Summary of the Invention

### Object to be Solved by the Invention

It is an object of the present invention to provide (A) a colorectal epithelial stem cell and/or a colorectal epithelial cell and (B) an extracellular matrix for use in the prevention or treatment of a human bowel disease.

### Means to Solve the Object

It was known before that three factors, Rspo1, Noggin, and EGF, are indispensable for culturing small intestinal cells (Sato, T., et al., Single Lgr5 stem cells build crypt-villus structures in vitro without a mesenchymal niche, Nature, 459, 262-265 (2009)). However, the combination of these three factors was not suitable for maintaining colorectal epithelial stem cells or the like. The present inventors have diligently studied under the circumstances of the above-described background art and have found the following facts (a) to (c), and the present invention has been accomplished:
(a) Colorectal epithelial stem cells can be efficiently isolated from colon tissue of a mammal, such as a mouse or a human being, by a specific treating process;
(b) It has been conventionally believed that in the case of in vitro culturing colorectal epithelial stem cells in a serum-free culture medium, in addition to basal medium components (a carbon source such as a saccharide, a nitrogen source such as an amino acid, and an inorganic salt), for example, gastrin, A83-01 (TGF-β1 receptor kinase inhibitor), SB202190 (p38 inhibitor), and B-27 supplement are essential components and that, in particular, B-27 supplement is particularly indispensable. However, colorectal epithelial stem cells or the like isolated from colon tissue of a mammal, such as a mouse or a human being, can be maintained or propagated for a long time in vitro, without using these components, even in a serum-free culture medium by adding serum albumin, Wnt3a, and r-spondin-1 to the culture medium, in addition to the basal medium components; and
(c) In vitro maintained or propagated colorectal epithelial stem cells or the like administered, by a developed specific method, into the colon of a mouse with acute colitis induced by administration of dextran sulfate sodium (DSS) are engrafted into the colon of the mouse and can enhance the recovery from the acute colitis.

That is, the present invention relates to:
1. (A) A colorectal epithelial stem cell and/or a colorectal epithelial cell and (B) an extracellular matrix for use in the prevention or treatment of a human bowel disease:
   wherein the cell is obtained by culturing a colorectal epithelial stem cell and/or a colorectal epithelial cell using a culture medium for *in vitro* culture of the colorectal epithelial stem cell and/or colorectal epithelial cell, and the extracellular matrix, the medium comprising serum albumin, Wnt3a, and r-spondin-1 as essential components and optionally further comprising epidermal growth factor (EGF), hepatocyte growth factor (HGF) and/or Noggin, and being serum-free;
   wherein the culture medium is B-27 supplement free;
   wherein the content of the serum albumin in the culture medium is in a range between 0.3% and 3% by weight;
   wherein the extracellular matrix is collagen type I gel; and
   wherein the cell is embedded in collagen type I gel and then cultured.

### Effect of the Invention

As disclosed herein, colorectal epithelial stem cells can be maintained or propagated for a long time in vitro, without using a serum culture medium. The colorectal epithelial stem cells maintain the sternness, and the colorectal epithelial stem cells administered to the colon of a mammal of which colorectal epithelium is damaged by, in particular, the method disclosed herein are well engrafted into the damaged colorectal epithelium and can construct colorectal epithelium that forms functionally and histologically normal and self-renewable colonic crypts. Accordingly, the colorectal epithelial stem cells cultured by the method disclosed herein can prevent and treat a bowel disease by being administered into the colon. In addition, though the composition of serum varies, since the method does not need such serum, the colorectal epithelial stem cells can be stably cultured with lower cost. Furthermore, according to the culture method disclosed herein, colorectal epithelial stem cells can be stably and reproducibly cultured from a minute quantity of colon tissue obtained during colonoscopy as a starting material, without requiring a large quantity of colon tissue, such as a surgical specimen. Furthermore, the isolation method disclosed herein can efficiently isolate colorectal epithelial stem cells from colon tissue of a mammal, and the colorectal epithelial stem cells isolated by the isolation method are suitable for the culture method disclosed herein.

### Brief Description of Drawings

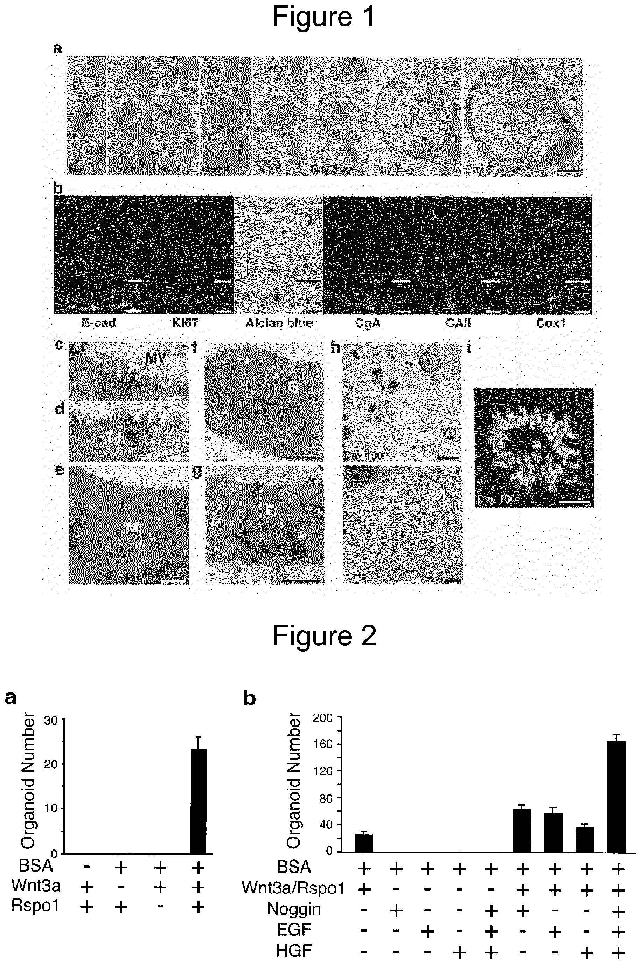

[Figure 1] (a) The figures show that a colonic crypt isolated from an adult mouse grows into a spherical cyst structure within 8 days of culturing of the colonic crypt cells by the culture method disclosed herein. The scale bar represents 50 µm. (b) The figures show the results of analysis by immunohistochemical staining or histochemical staining of colonic organoids formed by culturing colonic crypt cells, on the 8th day of the culturing. All cells constructing the colonic organoid were positive for E-cadherin staining (E-cad, green). Some cells were also positive for Ki67 staining (Ki67, green), alcian blue staining (Alcian blue, blue), CgA staining (CgA, green), CAII (CA-II) staining (CAII, green), and Cox1 staining (Cox1, green). High-magnification images of the regions within squares are shown in the lower side of the figures. Other than alcian blue staining, the nuclei were stained with DAPI (blue). The scale bar in the upper column represents 50 µm, and that in the lower column represents 10 µm. (c to g) The figures show the results of analysis of colonic organoids on the 8th day of the culturing with a transmission electron microscope. Microvilli, a structure specific to epithelial cells (c, indicated by "MV" in the figure), intercellular junction (tight junction) (d, indicated by "TJ" in the figure), a cell in the mitotic phase showing chromosome condensation (e, indicated by "M" in the figure), a goblet cell including mucous granules (f, indicated by "G" in the figure), and an enteroendocrine cell including electron-dense granules (g, indicated by "E" in the figure) were observed. The scale bars c and d each represent 0.5 µm, and the scale bars e to g each represent 5 µm. (h) The figures show the propagation of colonic organoids by subculture observed with a transmission electron microscope. The upper column is a low-magnification image of the colonic organoids on the 180th day of the culturing, and the lower column is a high-magnification image of a typical colonic organoid thereof. The scale bar in the upper column represents 500 µm, and that in the lower column represents 50 µm. (i) The figure shows the karyotype of a mitotic phase cell isolated from the colonic organoid cultured for 180 days and shows that the cell has a normal karyotype (2n = 40 chromosomes). The scale bar represents 10 µm. Note that since the actual figures obtained by the inventors included color fluorescences, the colors are referred to in the "Brief Description of Drawings" of the present application. However, in the black-and-white drawings attached to the specification of the present application, alcian blue staining appears in a blackish color, and other stainings appear in a whitish color.
[Figure 2] The graphs show the results of investigation for the effects of six factors (BSA, Wnt3a, Rspo1, Noggin, EGF, and HGF), added to the culture medium disclosed herein, on the propagation of colonic organoids. The number of formed colonic organoids was counted on the 8th day of the culturing of seeded 2000 colonic crypt cells in the presence of each combination of the factors shown in the graphs. The error bars represent SEM: n = 3.

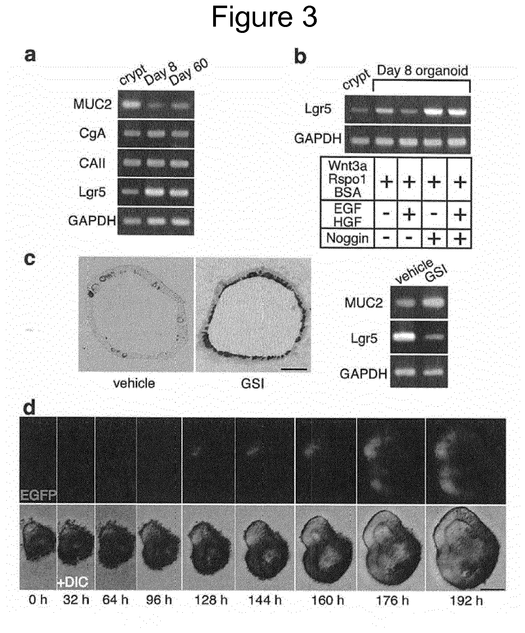

[Figure 3] (a) The figures show the results of RT-PCR analysis of four genes (MUC2, CgA, CAII, and Lgr5) expressed in colonic crypts (crypt) immediately after the isolation and in colonic organoids on the 8th day (Day 8) and the 60th day (Day 60) of the culturing. The GAPDH gene was used as a control. (b) The figures show the results of RT-PCR analysis of expression of the Lgr5 gene when colonic organoids were cultured for 8 days (Day 8) in the presence of various combinations of six factors (Wnt3a, Rspo1, BSA, EGF, HGF, and Noggin) shown under the figures. Colonic crypts (crypt) immediately after the isolation and the GAPDH gene were used as controls. (c) The figures show that a Notch signal is involved in in vitro maintenance of ability of the colonic organoids of the present invention for being undifferentiated. The figures on the left show the results of alcian blue staining of colonic organoids on the 4th to 8th days of the culturing treated with γ-secretase inhibitor (GSI) LY-411,575 (indicated as "GSI") or a solvent alone as a control (indicated as "Vehicle"). The scale bar represents 50 µm. The figures on the right show the results of RT-PCR analysis of expression of Muc2 and Lgr5 genes by treating colonic organoids with GSI or a solvent alone on the 4th to 8th days of the culturing. The GAPDH gene was used as a control. The experiment by RT-PCR was separately performed another two times, and similar results were obtained. (d) The figures show the manner of growth of a colonic crypt isolated from an Lgr5-EGFP-ires-CreERT2 mouse, over 192 hours. The upper column is fluorescent images of EGFP, and the lower column is merged images of the EGFP fluorescent images and respective DIC images.

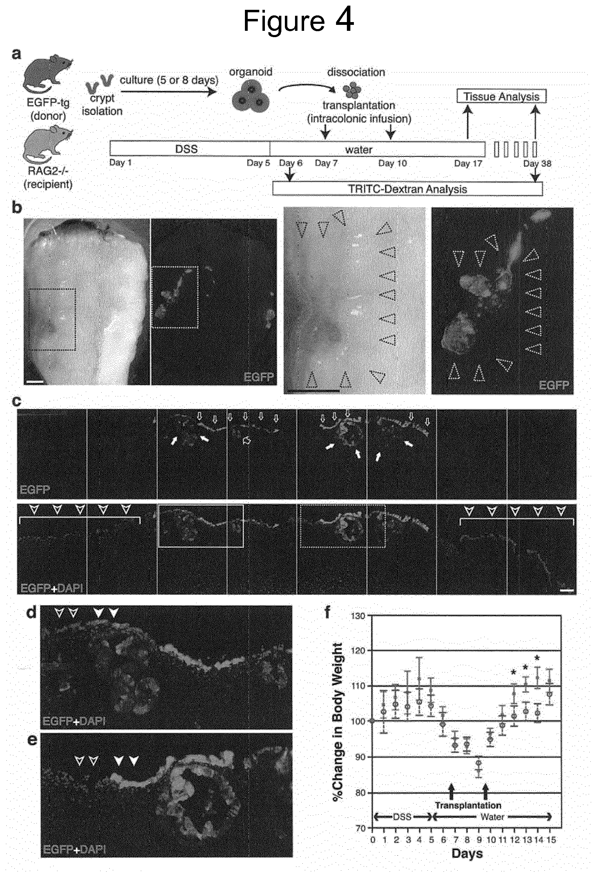

[Figure 4] (a) The diagram schematically illustrates a method of transplanting cells derived from colonic organoids of the present invention into a mouse. (b) The figures show the colon tissue on the 6th day from the transplantation of the cells derived from the colonic organoids of the present invention. Low-magnification stereomicroscopic and fluorescent images are shown on the left. The high-magnification images of the square regions surrounded with dotted lines in the left figures are shown on the right. In the stereomicroscopic image on the right, the arrowheads show the recessed region surrounded by edematous mucous membrane. The cells derived from the transplanted colonic organoids are detected as the fluorescence of EGFP (EGFP⁺). The stereomicroscopic image and the fluorescence microscopic image of the same visual field reveal that the EGFP⁺ region at which a graft was engrafted coincides with the recessed damaged region. These images were obtained with different microscopes such that the lumenal side is upside down, and therefore the outlines of the tissue do not completely agree with each other. The scale bar represents 1 mm. (c) The figures show the results of histological analysis of the EGFP⁺ region shown in figure 4b. Shown are the fluorescent images of EGFP (upper column) and merged images of the EGFP fluorescent images and respective DAPI staining images (lower column). (d) and (e) High-magnification images of the regions surrounded by a solid line and a dotted line in figure 4c are respectively shown as figures (d) and (e). The scale bar represents 100 µm. (f) The graph shows changes in body weight of mice when RAG2^{-/-} mice were fed with 3% DSS for 5 days and were subjected on the 7th and 10th days to transplantation (n = 6) or sham transplantation (N = 6) of cells derived from colonic organoids of the present invention. The mice were slaughtered on the 16th day, and transplantation efficiency was evaluated. The body weights of the mice transplanted with EGFP⁺ (square, n = 4) and the control mice received sham transplantation (white circle, n = 6) on each day are shown as proportions (%) to the respective body weights of the mice before the administration of DSS. The error bars represent standard errors (SEM), and the symbol * represents p < 0.05.

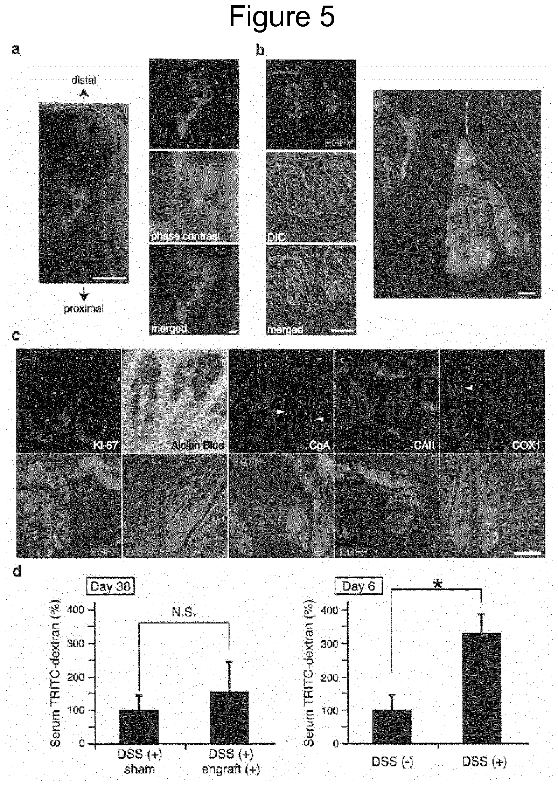

[Figure 5] (a) The figures show the results of analysis of the colorectal epithelium of the recipient with a fluorescence microscope at the 4th week after transplantation of the cells derived from colonic organoids of the present invention. The region surrounded by the dotted line is a distal terminal. High-magnification images of this region are shown on the right: EGFP fluorescent image (upper column), phase difference image (middle column), and their merged images (lower column). The scale bar in the left figure represents 500 µm, and that in the right figures represents 100 µm. (b) The figures show the results of immunohistochemical staining of the transplantation site with an anti-GFP antibody. Shown on the left are EGFP fluorescent image (upper column, "EGFP"), DIC image (middle column, "DIC"), and their merged images (lower column, Merged). The scale bar represents 50 µm. A high-magnification image of the merged image is shown on the right. The scale bar represents 10 µm. (c) The figures show the results of immunohistochemical staining or histochemical staining of serial sections of the transplanted tissue. In addition to the immunohistochemical staining or histochemical staining as in Figure 1b, stain-histochemical staining using an anti-GFP antibody was also performed. In the upper column, shown are images of Ki67⁺ cells (Ki67, red), goblet cells (Alcian blue, blue), enteroendocrine cells (CgA, red [in particular, indicated by arrowheads]), colonocytes (CAII, red), and tuft cells (Cox1, red [in particular, indicated by an arrowhead]). These images, excepting the alcian blue staining image, are merged images with nuclear staining images using DAPI (blue). The lower column shows staining results using an anti-GFP antibody (green) with the same visual field and focal point as those in the upper column. The scale bar represents 50 µm. (d) The graphs show the results of permeability assay using TRITC-dextran. The left graph shows the proportion (%) of the TRITC-dextran level in blood (blood TRITC level) of the transplantation group (DSS+, engraft [transplantation]+ group, n = 4) based on the assumption that the blood TRITC level of the sham transplantation group (DSS+, sham [sham] group, n = 6) is 100 (%) . As controls, RAG2^{-/-} mice with DSS-induced colitis (DSS+ group, n = 6) and RAG2^{-/-} mice without DSS-induced colitis (DSS- group, n = 6) were used without being subjected to transplantation. Permeability assay performed on the 6th day of the acute phase showed an increase of the blood TRITC level in the colitis-induced group as a reflection of the damage of mucous membrane. The right graph shows the proportion (%) of the blood TRITC level of the DSS+ group based on the assumption that the blood TRITC level of the DSS- group is 100 (%) . The error bar represents SEM, the symbol * represents p < 0.05, and N.S. denotes insignificance.

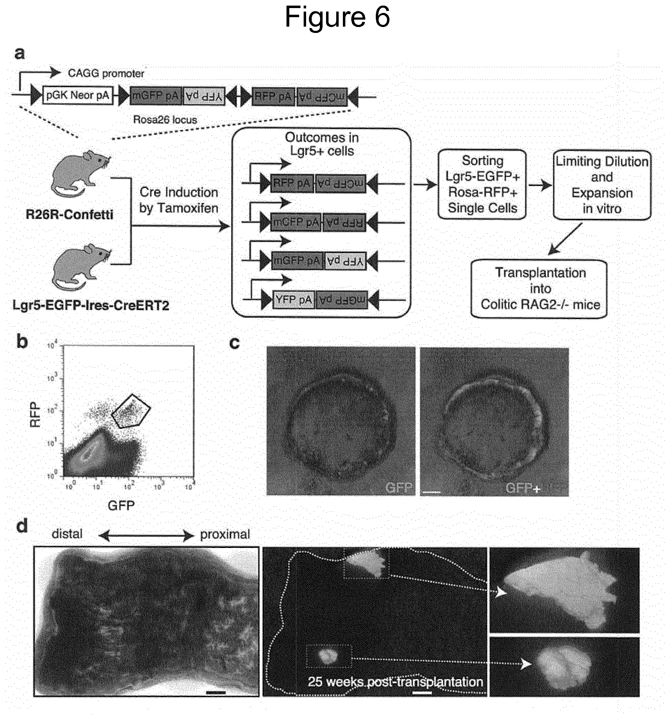

[Figure 6] (a) The diagram schematically illustrates an experimental method for propagating single colon stem cells by the culture method disclosed herein and visualizing the graft engrafted after transplantation. (b) The figure shows the result of FACS analysis of the colonocytes derived from an R26R-Confetti mouse mated with an Lgr5-EGFP-ires-CreERT2 mouse, 3 days after induction of Cre recombinase expression. The region surrounded by a solid line is EGFP⁺ cell population and RFP⁺ cell population. The cells (Lgr5-EGFP⁺/Confetti-RFP⁺ stem cells) present in this region were sorted and were used in the subsequent culture. (c) The figures show a colonic organoid on the 6th day grown from the sorted single Lgr5-EGFP⁺/Confetti-RFP⁺ stem cells. The fluorescent image of EGFP is shown on the left, and the fluorescent image of RFP on the right. The scale bar represents 50 µm. (d) The figures show the colon of a recipient at the 25th week after transplantation. The phase difference image is shown on the left, and the fluorescent image and the enlarged images of the regions surrounded by dotted lines are shown on the right. The scale bar represents 1 mm.

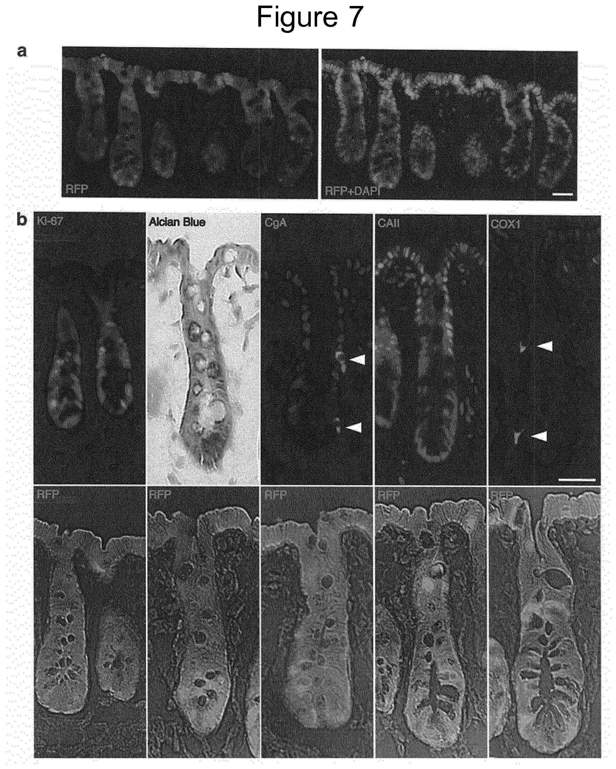

[Figure 7] (a) The figures show the results of immunohistochemical staining of RFP⁺ grafts using an anti-RFP antibody at 25 weeks after the second transplantation. Shown are an RFP fluorescent image at the transplantation site (left figure) and a merged image of the RFP fluorescent image and DIC (right figure). The scale bar represents 50 µm. (b) The figures show the results of immunohistochemical staining or histochemical staining of serial sections of the transplanted tissue. In addition to the immunohistochemical staining or histochemical staining as in Figure 1b, stain-histochemical staining using an anti-RFP antibody was also performed. In the upper column, shown are images of Ki67⁺ cells (Ki67, green), goblet cells (Alcian blue, blue), enteroendocrine cells (CgA, green [in particular, indicated by arrowheads]), colonocytes (CAII, green), and tuft cells (Cox1, green [in particular, indicated by arrowheads]). These images, excepting the alcian blue staining image, are merged images with nuclear staining images using DAPI (blue). The lower column shows staining results using an anti-RFP antibody (red) with the same visual field and focal point as those of the upper column. The scale bar represents 50 µm.

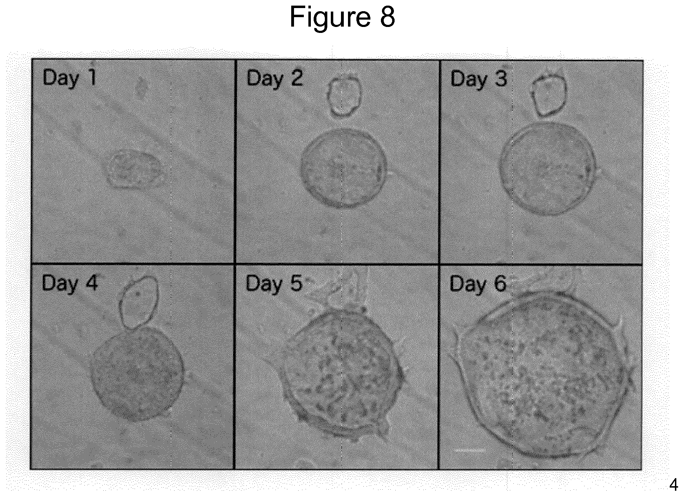

[Figure 8] The figures show that the colonic crypt cells can be cultured in vitro even in the presence of arbitrary components (insulin, transferrin, a selenite, sodium pyruvate, N-acetylcysteine, a cholera toxin, a nucleoside mixture solution, or ceruloplasmin) other than EGF, HGF, and Noggin. The scale bar represents 50 µm.

### Mode of Carrying Out the Invention

### 1. Culture medium for in vitro culture of a colorectal epithelial stem cell and/or a colorectal epithelial cell

The "culture medium for in vitro culture of a colorectal epithelial stem cell and/or a colorectal epithelial cell" disclosed herein (hereinafter, also simply referred to as "the culture medium disclosed herein") may be any culture medium that contains serum albumin, Wnt3a, and r-spondin-1 (hereinafter, these three components are also collectively simply referred to as "essential three components") in addition to basal medium components (a carbon source such as a saccharide, a nitrogen source such as an amino acid, and an inorganic salt). The use of a culture medium containing such components in the culture of colorectal epithelial stem cells allows the colorectal epithelial stem cells to be maintained or propagated for a long time in vitro, without using a serum culture medium and also allows the colorectal epithelial stem cells to maintain the sternness. In addition, though the composition of serum varies, since the present invention does not need such serum, the colorectal epithelial stem cells can be stably cultured with lower cost. Throughout the specification, the term "colorectal epithelial stem cell(s)" of the present invention includes cell mixtures containing colorectal epithelial stem cells and colorectal epithelial cells. The content proportion of the colorectal epithelial stem cells in such a cell mixture can be, for example, 5% or more, 10% or more, 20% or more, 30% or more, 40% or more, 50% or more, 60% or more, or 80% or more.

The serum albumin may be any albumin contained in serum of a mammal. Such serum albumin may be any of albumin purified from serum of a mammal, chemically or biochemically synthesized albumin, or commercially available albumin (for example, BSA manufactured by Sigma-Aldrich Corporation). The serum albumin may be derived from any mammal, and preferred examples thereof include human serum albumin (HSA) and bovine serum albumin (BSA), and more preferred is BSA. It is also preferred to use serum albumin derived from a mammal of the same species as that of the mammal from which the colorectal epithelial stem cells to be cultured are derived.

The content of the serum albumin in the culture medium disclosed herein is within a range of 0.3% or more and 3% or less.

The Wnt3a may be any protein that is encoded by a mammal WNT3A gene and may be, for example, Wnt3a produced by Wnt3-producing cells such as Paneth cells, Wnt3a produced by cells generated by genetic engineering so as to express the mammal WNT3A gene, chemically or biochemically synthesized Wnt3a, or commercially available Wnt3a (for example, Wnt3a manufactured by R&D Systems, Inc.). The origin of Wnt3a may be any mammal. Preferred examples thereof include human Wnt3a (hWnt3a) and mouse Wnt3a (mWnt3a), and more preferred is mWnt3a. It is also preferred to use Wnt3a derived from a mammal of the same species as that of the mammal from which the colorectal epithelial stem cells to be cultured are derived.

The content of Wnt3a in the culture medium disclosed herein is not particularly limited as long as it can be used for culturing colorectal epithelial stem cells and is preferably, for example, 3 mg/mL or less (e.g., 500 µg/mL or less, 100 µg/mL or less, 30 µg/mL or less, 3 µg/mL or less, or 1 µg/mL or less), as the final concentration in the culture medium disclosed herein. The final concentration can be, for example, 300 pg/mL or more (e.g., 1 ng/mL or more, 3 ng/mL or more, 10 ng/mL or more, or 30 ng/mL or more). The final concentration is more preferably 30 ng/mL or more.

The r-spondin-1 (Rspo1) may be any protein that is encoded by a mammal RSPO1 gene and may be, for example, Rspo1 produced by Rspo1-producing cells such as cells generated by genetic engineering so as to express the mammal RSPO1 gene, chemically or biochemically synthesized Rspo1, or commercially available Rspo1 (for example, R-Spondin 1 manufactured by R&D Systems, Inc.). The origin of Rspo1 may be any mammal. Preferred examples thereof include human Rspo1 (hRspo1) and mouse Rspo1 (mRspo1), and more preferred is mRspo1. It is also preferred to use Rspo1 derived from a mammal of the same species as that of the mammal from which the colorectal epithelial stem cells to be cultured are derived.

The content of Rspo1 in the culture medium disclosed herein is not particularly limited as long as it can be used for culturing colorectal epithelial stem cells and is preferably, for example, 5 mg/mL or less (e.g., 1 mg/mL or less, 200 µg/mL or less, 40 µg/mL or less, 5 µg/mL or less, or 1 µg/mL or less), as the final concentration in the culture medium disclosed herein. The final concentration can be, for example, 500 pg/mL or more (e.g., 5 ng/mL or more, 50 ng/mL or more, or 250 ng/mL or more).

Preferred examples of the basal medium components include carbon sources such as saccharides that can be assimilated by colorectal epithelial stem cells to be cultured, nitrogen sources such as amino acids that can be digested by the colorectal epithelial stem cells, and inorganic salts. The culture medium containing such basal medium components may be one known to a person skilled in the art or a commercially available one (for example, culture media manufactured by Invitrogen), and specific examples thereof include Minimum Essential Medium (MEM), Basal Medium Eagle (BME), Dulbecco's Modified Eagle Medium (DMEM), Eagle's minimal essential medium (EMEM), Iscove's Modified Dulbecco's Medium (IMDM), Glasgow's MEM (GMEM), Ham's F12 Medium (F12), DMEM/F12, RPMI1640, Brinster's BMOC-3 Medium (BMOC-3), CMRL-1066, Leibovitz's L-15 medium (L-15 medium), McCoy's 5A, Media 199, MEM αMedia, MCDB105, MCDB131, MCDB153, MCDB201, Williams' medium E, Advanced MEM, Advanced DMEM, Advanced DMEM/F-12, and Advanced RPMI1640. Advanced DMEM/F-12 (manufactured by Invitrogen) can be preferably used as the culture medium disclosed herein containing the basal medium components. Examples of the amino acid include essential amino acids and non-essential amino acids. In particular, glutamine, which is a non-essential amino acid, can be preferably used.

The culture medium disclosed herein may contain the basal medium components and the essential three components only or, from the viewpoint of increasing the propagation efficiency of colorectal epithelial stem cells, may preferably further contain, in addition to these components, one or more components selected from the group consisting of epidermal growth factor (EGF), hepatocyte growth factor (HGF), Noggin, insulin, transferrin, selenites, sodium pyruvate, antioxidants, cholera toxins, nucleic acids, ceruloplasmin, vitamins, and serum (hereinafter, these 13 components may be referred to as "arbitrary 13 components"). In particular, the culture medium more preferably contains at least one or more selected from the group consisting of EGF, HGF, and Noggin and preferably at least these three components. Still more preferably, the culture medium contains one or more of the group consisting of EGF, HGF, and Noggin or preferably three of these components and also contains one or more components selected from the group consisting of insulin, transferrin, selenites, sodium pyruvate, antioxidants, cholera toxins, nucleic acids, and ceruloplasmin. The culture medium disclosed herein may contain serum, but from the viewpoint of having higher effects of the present invention, the culture medium preferably does not contain serum.

The EGF may be any protein that is encoded by a mammal EGF gene and may be, for example, EGF produced by EGF-producing cells such as cells generated by genetic engineering so as to express the mammal EGF gene, chemically or biochemically synthesized EGF, or commercially available EGF (for example, EGF manufactured by PeproTech, Inc.). The origin of EGF may be any mammal. Preferred examples thereof include human EGF (hEGF) and mouse EGF (mEGF), and more preferred is mEGF. It is also preferred to use EGF derived from a mammal of the same species as that of the mammal from which the colorectal epithelial stem cells to be cultured are derived.

The content of EGF in the culture medium disclosed herein is not particularly limited as long as it can be used for culturing colorectal epithelial stem cells and is preferably, for example, 200 µg/mL or less (e.g., 40 µg/mL or less, 8 µg/mL or less, 2 µg/mL or less, 200 ng/mL or less, or 40 ng/mL or less), as the final concentration in the culture medium disclosed herein. The final concentration can be, for example, 1 pg/mL or more (e.g., 8 pg/mL or more, 100 pg/mL or more, 1 ng/mL or more, or 10 ng/mL or more). The concentration is more preferably, for example, within a range of 1 ng/mL or more and 40 ng/mL or less.

The HGF may be any protein that is encoded by a mammal HGF gene and may be, for example, HGF produced by HGF-producing cells such as cells generated by genetic engineering so as to express the mammal HGF gene, chemically or biochemically synthesized HGF, or commercially available HGF (for example, HGF manufactured by R&D Systems, Inc.). The origin of HGF may be any mammal. Preferred examples thereof include human HGF (hHGF) and mouse HGF (mHGF) , and more preferred is mHGF. It is also preferred to use HGF derived from a mammal of the same species as that of the mammal from which the colorectal epithelial stem cells to be cultured are derived.

The content of HGF in the culture medium disclosed herein is not particularly limited as long as it can be used for culturing colorectal epithelial stem cells and is preferably, for example, 500 µg/mL or less (e.g., 100 µg/mL or less, 20 µg/mL or less, 4 µg/mL or less, 500 ng/mL or less, or 100 ng/mL or less), as the final concentration in the culture medium disclosed herein. The final concentration can be, for example, 50 pg/mL or more (e.g., 500 pg/mL or more, 5 ng/mL or more, or 25 ng/mL or more). The concentration is more preferably, for example, within a range of 5 ng/mL or more and 100 ng/mL or less.

The Noggin may be any protein that is encoded by a mammal NOG gene and may be, for example, Noggin produced by Noggin-producing cells such as cells generated by genetic engineering so as to express the mammal NOG gene, chemically or biochemically synthesized Noggin, or commercially available Noggin (for example, Noggin manufactured by R&D Systems, Inc.). The origin of Noggin may be any mammal. Preferred examples thereof include human Noggin (hNoggin) and mouse Noggin (mNoggin), and more preferred is mNoggin. It is also preferred to use Noggin derived from a mammal of the same species as that of the mammal from which the colorectal epithelial stem cells to be cultured are derived.

The content of Noggin in the culture medium disclosed herein is not particularly limited as long as it can be used for culturing colorectal epithelial stem cells and is preferably, for example, 500 µg/mL or less (e.g., 100 µg/mL or less, 20 µg/mL or less, 4 µg/mL or less, 500 ng/mL or less, or 100 ng/mL or less), as the final concentration in the culture medium disclosed herein. The final concentration can be, for example, 50 pg/mL or more (e.g., 500 pg/mL or more, 5 ng/mL or more, or 25 ng/mL or more). The concentration is more preferably, for example, within a range of 5 ng/mL or more and 100 ng/mL or less.

The insulin may be any protein that is encoded by a mammal insulin gene and may be, for example, insulin produced by insulin-producing cells such as cells generated by genetic engineering so as to express the mammal insulin gene, chemically or biochemically synthesized insulin, or commercially available insulin. The origin of insulin may be any mammal. Preferred examples thereof include human insulin and mouse insulin. It is also preferred to use insulin derived from a mammal of the same species as that of the mammal from which the colorectal epithelial stem cells to be cultured are derived. The content of insulin in the culture medium disclosed herein is not particularly limited as long as it can be used for culturing colorectal epithelial stem cells and
is preferably, for example, within a range of 1 to 100 µg/ml or within a range of 5 to 20 µg/ml as the final concentration.

The transferrin may be any protein that is encoded by a mammal transferrin gene and may be, for example, transferrin produced by transferrin-producing cells such as cells generated by genetic engineering so as to express the mammal transferrin gene, chemically or biochemically synthesized transferrin, or commercially available transferrin. The origin of transferrin may be any mammal. Preferred examples thereof include human transferrin and mouse transferrin. It is also preferred to use transferrin derived from a mammal of the same species as that of the mammal from which the colorectal epithelial stem cells to be cultured are derived. The content of insulin when transferrin is contained in the culture medium disclosed herein is not particularly limited as long as it can be used for culturing colorectal epithelial stem cells and is preferably, for example, within a range of 0.55 to 55 µg/ml or within a range of 2.75 to 11 µg/ml as the final concentration.

Preferred examples of the selenite include sodium selenite. The selenite may be a commercially available one. The content of the selenite in the culture medium disclosed herein is not particularly limited as long as it can be used for culturing colorectal epithelial stem cells and is preferably, for example, within a range of 0.67 to 67 ng/ml or within a range of 3.35 to 13.4 ng/ml as the final concentration.

Meanwhile, insulin-transferrin-selenite mixture solution (ITS) manufactured by Invitrogen is a preferred example of commercially available product containing all of insulin, transferrin, and selenite.

The sodium pyruvate may be a commercially available one such as that manufactured by GIBCO RBL. The content of the selenite in the culture medium disclosed herein is not particularly limited as long as it can be used for culturing colorectal epithelial stem cells and is preferably, for example, within a range of 11 to 1100 µg/ml or within a range of 55 to 220 µg/ml as the final concentration.

As the antioxidant, for example, N-acetylcysteine can be preferably used. The antioxidant may be a commercially available one. For example, N-acetylcysteine manufactured by Nacalai Tesque, Inc. can be used. The content of the antioxidant in the culture medium disclosed herein is not particularly limited as long as it can be used for culturing colorectal epithelial stem cells and is preferably, for example, in a case of N-acetylcysteine, within a range of 10 to 1000 µM or within a range of 50 to 200 µM as the final concentration.

The cholera toxin may be any cholera toxin (also referred to as cholera enterotoxin). The cholera toxin may be a commercially available one such as that manufactured by Wako Pure Chemical Industries, Ltd. The content of the cholera toxin in the culture medium disclosed herein is not particularly limited as long as it can be used for culturing colorectal epithelial stem cells and is preferably, for example, within a range of 20 to 2000 ng/ml or within a range of 100 to 400 ng/ml as the final concentration.

The nucleic acid may be any nucleoside such as ribonucleoside or deoxyribonucleoside. The nucleoside may be a commercially available one such as that manufactured by Sigma-Aldrich Corporation. The content of the nucleoside in the culture medium disclosed herein is not particularly limited as long as it can be used for culturing colorectal epithelial stem cells and is preferably, for example, within a range of 10 to 1000 µM or within a range of 50 to 200 µM as the final concentration.

The vitamin may be any vitamin and may be a commercially available one such as that manufactured by GIBCO RBL. The vitamin can be used within a concentration that does not impair the effects of the present invention. In the present invention, the vitamin expediently also includes known vitamin-like materials.

The ceruloplasmin may be any protein that is encoded by a mammal ceruloplasmin gene and may be, for example, ceruloplasmin produced by ceruloplasmin-producing cells such as cells generated by genetic engineering so as to express the mammal ceruloplasmin gene, chemically or biochemically synthesized ceruloplasmin, or commercially available ceruloplasmin such as ceruloplasmin manufactured by Sigma-Aldrich Corporation. The origin of ceruloplasmin may be any mammal. Preferred examples thereof include human ceruloplasmin and mouse ceruloplasmin. It is also preferred to use ceruloplasmin derived from a mammal of the same species as that of the mammal from which the colorectal epithelial stem cells to be cultured are derived. The content of the ceruloplasmin in the culture medium disclosed herein is not particularly limited as long as it can be used for culturing colorectal epithelial stem cells and is preferably, for example, within a range of 3.8 to 380 nM or within a range of 19 to 76 nM.

The serum may be any mammal serum, and a preferred example thereof is fetal calf serum (FCS). The serum may be a commercially available one such as that manufactured by Gibco or that manufactured by Thermo Scientific HyClone. The serum is preferably used within a concentration that does not impair the effects of the present invention.

The culture medium disclosed herein may contain an arbitrary component other than the arbitrary 13 components. Preferred examples of the arbitrary component include antibiotics such as penicillin, streptomycin, and gentamycin; Rho kinase inhibitors such as Y-27632 (Calbiochem (registered trademark), manufactured by MERCK); and extracellular matrices such as collagen.

The culture medium disclosed herein can be prepared by, for example, a method in which the basal medium components, the essential three components, and optional components are added to a liquid solvent such as water or PBS or a method in which the essential three components and optional components are added to a liquid culture medium containing the basal medium components.

### 2. Method of in vitro culturing a colorectal epithelial stem cell and/or colorectal epithelial cell

The "method of in vitro culturing colorectal epithelial stem cells" disclosed herein (hereinafter, also simply referred to as "culture method disclosed herein") is not particularly limited as long as the method comprises a step Z of culturing colorectal epithelial stem cells using the culture medium disclosed herein and an extracellular matrix. Examples of the extracellular matrix include various types of collagen, types I to VIII, and matrigel. Among them, from the viewpoint of not containing materials that are produced by cultured cancer cells, such as sarcoma cells, and having high safety on transplantation into a mammal, collagen is preferred. In particular, from the viewpoint of capable of selecting colorectal epithelial stem cells more suitable for transplantation, collagen type I (collagen I) is more preferred. The various types of collagen and the matrigel may be commercially available ones (for example, collagen manufactured by Nitta Gelatin Inc. and matrigel manufactured by BD Biosciences). Matrigel is an extracellular matrix extracted from Engelbreth-Holm-Swarm (EHS) mouse sarcoma cells, and contains laminin, collagen IV, heparan sulfate proteoglycan, and entactin/nidogen as main components and also contains other growth factors that are naturally produced in EHS sarcoma cells, such as TGF-β, insulin-like growth factor, fibroblast growth factor, and a tissue plasminogen activator.

According to the culture method disclosed herein, colorectal epithelial stem cells can be maintained or propagated in vitro for a long time (preferably 1 month or more, more preferably 3 months or more, still more preferably 6 months or more, and most preferably 1 year or more), without using a serum culture medium. The colorectal epithelial stem cells maintain the sternness, and the colorectal epithelial stem cells administered to the colon of a mammal of which colorectal epithelium is damaged are engrafted into the colorectal epithelium and can construct a colorectal epithelium that forms a functionally and histologically normal and self- renewable colonic crypt. In addition, according to the culture method disclosed herein, colorectal epithelial stem cells can be stably and reproducibly cultured from a minute quantity of colon tissue obtained during colonoscopy as a starting material, without requiring a large quantity of colon tissue, such as a surgical specimen.

The colorectal epithelial stem cellsused in the step Z may be any colorectal epithelial stem cells derived from a mammal, and preferred examples thereof include colorectal epithelial stem cells collected from colon tissue of a mammal. In particular, more preferred examples include colorectal epithelial stem cells isolated from the colon tissue, and still more preferred examples include colorectal epithelial stem cells isolated by the isolation method described in the section "4." below.

The culture method in the step Z is not particularly limited as long as the method can maintain or propagate colorectal epithelial stem cells using the culture medium disclosed herein and an extracellular matrix, and examples thereof include a method of culturing colorectal epithelial stem cells under conditions allowing the colorectal epithelial stem cells to proliferate in coexistence of the culture medium disclosed herein, an extracellular matrix, and the colorectal epithelial stem cells. In particular, a preferred example is a method of culturing colorectal epithelial stem cells adhered to an extracellular matrix or colorectal epithelial stem cells embedded in an extracellular matrix (preferably collagen and more preferably collagen type I), in the culture medium disclosed herein. A more preferred example is a method of culturing colorectal epithelial stem cells three-dimensionally embedded in an extracellular matrix, prepared by suspending the colorectal epithelial stem cells in a solution of the extracellular matrix (preferably a collagen solution and more preferably a collagen type I solution) and then semi-solidifying (e.g., gelation or solation) the suspension, in the culture medium disclosed herein. Particularly preferred examples include the culture methods described below. The culture of colorectal epithelial stem cells embedded in an extracellular matrix can form a tissue structure called organoid, in which functional cells, such as absorptive epithelial cells and mucus-producing cells, are arranged similar to in vivo tissue and is therefore particularly preferred. The organoid can exhibit an excellent prophylactic or therapeutic effect against a bowel disease when it is administered or transplanted into the colon of a mammal.

The culture conditions in the step Z are not particularly limited as long as colorectal epithelial stem cells can be maintained or propagated. For example, the culture temperature is within a range of 30°C to 40°C, preferably 36°C to 38°C, and more preferably 37°C. From the viewpoint of more suitably maintaining or propagating colorectal epithelial stem cells, culture medium replacement and subculture are preferably performed at suitable times. The frequency of culture medium replacement is not particularly limited as long as colorectal epithelial stem cells can be maintained or propagated, and can be, for example, once every 1 to 5 days and preferably once every 3 days. The frequency of subculture is not particularly limited as long as colorectal epithelial stem cells can be maintained or propagated, and can be suitably performed at the time that the population of colorectal epithelial stem cells (preferably organoid) has become large. The frequency can be, for example, once every 4 to 12 days and preferably once every 6 to 10 days. The method of subculture is not particularly limited as long as colorectal epithelial stem cells can be maintained or propagated, and examples thereof include a method in which colorectal epithelial stem cells separated from the extracellular matrix are brought to coexist with a fresh extracellular matrix. In particular, in the case of using a semi-solidified extracellular matrix (preferably collagen), preferred examples of the subculture method include a method in which colorectal epithelial stem cells separated by degradation of the extracellular matrix with a degrading enzyme (preferably, e.g., collagenase) are embedded in a fresh extracellular matrix. In particular, a more preferred example of the subculture method comprises the following steps m to t:
step m: a step of removing the culture medium disclosed herein;
step n: a step of collecting extracellular matrix (preferably collagen and more preferably collagen type I) containing colorectal epithelial stem cells;
step o: a step of adding the extracellular matrix containing colorectal epithelial stem cells to a buffer (preferably DMEM) containing an extracellular matrix-degrading enzyme (preferably collagenase and more preferably collagenase XI) and degrading the extracellular matrix by enzymatic treatment under conditions to allow the degrading enzyme to exhibit the degradation activity;
step p: a step of washing the extracellular matrix containing colorectal epithelial stem cells with a buffer (preferably Hanks' Balanced Salt Solution (HBSS));
step q: a step of adding the colorectal epithelial stem cells to a protein-degrading enzyme (preferably trypsin) solution and dispersing each cell of the colorectal epithelial stem cells by enzymatic treatment under conditions to allow the protein-degrading enzyme to exhibit the degradation activity;
step r: a step of washing the colorectal epithelial stem cells with DMEM;
step s: a step of suspending the washed colorectal epithelial stem cells in an extracellular matrix solution (preferably a collagen solution and more preferably collagen type I solution) and then semi-solidifying (gelation or solation) the suspension to three-dimensionally embed the colorectal epithelial stem cells in the extracellular matrix; and
step t: a step of adding the culture medium disclosed herein to the embedded product.

Particularly preferred examples of the subculture method include the methods described below.

The "colorectal epithelial stem cell" in the specification may be any colorectal epithelial stem cell having tissue-regeneration ability, and a preferred example thereof is an Lgr5 positive (Lgr5⁺) colorectal epithelial stem cell. Whether such colorectal epithelial stem cells maintain sternness or not can be determined by investigating whether such colorectal epithelial stem cells have tissue-regeneration ability or not, that is, whether, when such colorectal epithelial stem cells are administered to the colon of a mammal of which colorectal epithelium is damaged, the colorectal epithelial stem cells are engrafted in the colon and regenerate colorectal epithelium tissue or not.

### 3. (A) Colorectal epithelial stem cell and/or colorectal epithelial cell and (B) extracellular matrix for use in the prevention or treatment of a human bowel disease

The (A) colorectal epithelial stem cell and/or colorectal epithelial cell and (B) extracellular matrix for use in the prevention or treatment of a human bowel disease (hereinafter, also simply referred to as "prophylactic or therapeutic agent of the present invention") comprises colorectal epithelial stem cells and/or a colorectal epithelial cells cultured by the culture method disclosed herein. Specific examples of the bowel disease include inflammatory bowel diseases such as ulcerative colitis and Crohn's disease, damaged intestinal tracts due to chemotherapy, and radiation enteritis. Administration of the prophylactic or therapeutic agent of the present invention causes engraftment of the colorectal epithelial stem cells in the colorectal epithelium to prevent or treat the damage of the colorectal epithelium. The "prophylactic or therapeutic effect on a bowel disease" in the specification includes prophylactic effect on a bowel disease, a therapeutic effect on a bowel disease, and prophylactic and therapeutic effects on a bowel disease. The prophylactic effect on a bowel disease includes an effect of preventing the onset of the bowel disease and an effect of retarding the onset of the bowel disease. The therapeutic effect on a bowel disease includes an effect of improving the symptom of the bowel disease and an effect of preventing or retarding worsening of the symptom of the bowel disease.

The colorectal epithelial stem cells in the prophylactic or therapeutic agent of the present invention may be any colorectal epithelial stem cells cultured by the culture method disclosed herein. In particular, from the viewpoint of obtaining more excellent prophylactic or therapeutic effect on a bowel disease, for example, colorectal epithelial stem cells that have formed organoids are preferred. In particular, colorectal epithelial stem cells that have formed organoids are more preferred. Furthermore, colorectal epithelial stem cells derived from the colon of a mammal to which the prophylactic or therapeutic agent of the present invention is administered are preferred, since suppression of the rejection reaction is not required.

The prophylactic or therapeutic agent of the present invention may be administered to any mammal. Preferred examples of the mammal in the specification include human, monkey, mouse, rat, hamster, guinea pig, bovine, pig, horse, rabbit, sheep, goat, cat, and dog. In particular, human is more preferred.

The prophylactic or therapeutic agent of the present invention may be administered by any method as long as a bowel disease can be prevented or treated, and examples of the method include enteral administration and intravenous administration. In particular, transanal enema administration to the colon is preferred. The dose of the prophylactic or therapeutic agent of the present invention varies depending on, for example, the type of the bowel disease, the degree of the symptom, and the body weight and age of the subject and is, for example, 1 × 10 to 1 × 10¹⁰ cells/kg (which may be administered in one to several times a day).

The prophylactic or therapeutic agent of the present invention may be prepared by any method and can be prepared by, for example, blending colorectal epithelial stem cells cultured by the culture method disclosed herein with pharmaceutically acceptable common ingredients for pharmaceuticals, e.g., a carrier, an excipient, a diluent, a pH buffer, a water-soluble solvent such as physiological saline, an isotonizing agent such as sodium chloride, glycerol, or D-mannitol, a stabilizer such as human serum albumin, a preservative such as methylparaben, and a local anesthetic such as benzyl alcohol. From the viewpoint of improving the efficiency of engraftment in the colorectal epithelium of a subject, the prophylactic or therapeutic agent of the present invention preferably contains extracellular matrix components such as various types of collagen, types I to VIII, and matrigel and more preferably matrigel. In addition, the prophylactic or therapeutic agent of the present invention may contain, in addition to a colorectal epithelial stem cell cultured by the culture method disclosed herein, another prophylactic or therapeutic agent for a bowel disease.

### 4. Method of isolating colorectal a epithelial stem cell and/or a colorectal epithelial cell

The "method of isolating a colorectal epithelial stem cell and/or a colorectal epithelial cell" disclosed herein (hereinafter, also simply referred to as "isolation method disclosed herein ") is not particularly limited as long as it comprises the following steps A to D in this order:
step A: a step of collecting a colon tissue containing a colorectal epithelial stem cell and/or a colorectal epithelial cell from the colon of a mammal;
step B: a step of digesting the colon tissue with collagenase, dispase, and a reducing agent;
step C: a step of homogenizing a tissue fragment of the digested colon tissue; and
step D: a step of subjecting the homogenized colon tissue to concentration gradient centrifugation to obtain a fraction containing a colorectal epithelial stem cell and/or a colorectal epithelial cell.

The isolation method disclosed herein can efficiently isolate a colorectal epithelial stem cell from the colon tissue of a mammal, and the colorectal epithelial stem cell isolated by this isolation method are suitable for the culture method disclosed herein.

The step A may be any step in which colon tissue containing a colorectal epithelial stem cell is collected from the colon of a mammal, and a preferred example thereof is a method of collecting colon tissue from the colon of a mammal using a forceps that is usually used in examination of tissue during colonoscopy. The amount of the colon tissue to be collected is not particularly limited, and an amount of a tissue of a diameter of about 5 to 6 mm is sufficient for the culture method disclosed herein. The isolation method disclosed herein preferably further comprises the following steps u to w in this order after the step A and before the step B:
step u: a step of immersing the collected colon tissue in a buffer (preferably HBSS) containing an antibiotic such as penicillin, streptomycin, or gentamycin, preferably in such a buffer cooled at 4°C on ice;
step v: a step of washing the colon tissue by shaking the colon tissue, preferably washing the colon tissue again after replacement of the buffer containing an antibiotic by shaking the colon tissue; and
step w: a step of separating the washed colon tissue from the buffer and cutting the colon tissue into a minced state, a chopped state,.

The step B may be any step in which the colon tissue is digested with collagenase, dispase, and a reducing agent. A preferred example of the collagenase is collagenase XI, and a preferred example of the reducing agent is dithiothreitol (DTT). A preferred example of the step B is a step of digesting the colon tissue by adding the colon tissue to a DMEM containing collagenase XI, dispase, DTT, and fetal bovine serum (FBS). Such digestion treatment is preferably performed at a temperature (preferably 37°C) allowing the collagenase and dispase to exhibit the enzymatic activity with shaking, from the viewpoint of improving the digestion efficiency.

The step C may be any step in which a tissue fragment of the digested colon tissue is homogenized. A preferred example of the method of homogenizing treatment is a method of homogenizing the colon tissue fragment by allowing the colon tissue fragment to pass through a syringe with a needle (preferably a syringe with a needle having a gauge of 6 to 21) multiple times. The isolation method disclosed herein preferably comprises a step of adding a DMEM containing sorbitol to the homogenized colon tissue and performing centrifugation before the step D. In this case, the pellet obtained by the centrifugation can be used as the "homogenized colon tissue" in the step D.

The step D may be any step in which a fraction containing a colorectal epithelial stem cell and/or a colorectal epithelial cell is obtained by subjecting the homogenized colon tissue to concentration gradient centrifugation. The concentration gradient centrifugation can be performed by a common method. A preferred example thereof is a method in which the homogenized colon tissue is suspended in a percoll solution and centrifugation is then performed. The pellet obtained by the concentration gradient centrifugation contains a large quantity of a colorectal epithelial stem cell (in particular, colonic crypts containing a colorectal epithelial stem cell) and, therefore, can be preferably used as a fraction containing the colorectal epithelial stem cell. The method of preparing the pellet preferably further comprises the following steps x and y in this order:
step x: a step of removing coarse tissue by suspending the pellet prepared in the step C in a DMEM containing sorbitol and allowing the suspension to pass through a mesh (preferably a mesh size of 300 µm); and
step y: a step of suspending the colorectal epithelial stem cell after the removal of coarse tissue in a DMEM containing sorbitol, centrifuging the suspension, and washing the resulting pellet.

Particularly preferred embodiments of the isolation method disclosed herein include the isolation methods described below.

### 5. Others

Also disclosed herein are the use of serum albumin, Wnt3a, and r-spondin-1 in production of a culture medium for in vitro culture of a colorectal epithelial stem cell, a method of preventing or treating a bowel disease comprising a step of administering the prophylactic or therapeutic agent of the present invention to a mammal, the use of a colorectal epithelial stem cell cultured by the culture method disclosed herein in production of an agent for preventing or treating a bowel disease, a method of transplanting a colorectal epithelial stem cell cultured by the culture method disclosed herein in the colon of a mammal, a method of producing a colorectal epithelial stem cell for being transplanted in the colon of a mammal comprising the culture method disclosed herein, and a prophylactic or therapeutic method of a bowel disease comprising a step of transplanting a colorectal epithelial stem cell cultured by the culture method disclosed herein in the colon of a mammal.

The present invention will now be more specifically described by examples, but the present invention is not limited to the following examples.

### Examples

### Example 1

### [Mouse]

In this example, the following mice were used.

C57BL/6-Ly5.2 RAG-2 knockout (RAG2^{-/-}) mice (produced by Taconic Farms, Inc. or Central Laboratories for Experimental Animals); EGFP transgenic mice expressing EGFP under control by a chicken β-actin promoter and a cytomegalovirus enhancer (Okabe, M., Ikawa, M., Kominami, K., Nakanishi, T. & Nishimune, Y., 'Green mice' as a source of ubiquitous green cells, FEBS Lett., 407, 313-319 (1997)); Lgr5-EGFP-IRES-CreERT2 mice (Barker, N., et al. , Identification of stem cells in small intestine and colon1 by marker gene Lgr5, Nature, 449, 1003-1007 (2007), available from Jackson Lab. as Stock No. 008875); and R26R-Confetti mice (Snippert, H.J., et al., Intestinal crypt homeostasis results from neutral competition between symmetrically dividing Lgr5 stem cells, Cell, 143, 134-144 (2010)).

### [Isolation of colonic crypt and TMDU protocol for 3D culture]

Colonic crypts were isolated from 7- to 9-week-old adult mice by the following procedure. First, the whole colon was collected, and the lumenal contents were washed out with a Hank's buffered salt solution (HBSS) containing 100 U/mL of penicillin, 100 µg/mL of streptomycin, and 50 µg/mL of gentamycin. These antibiotics were added to all solutions used in the following steps. The colon tissue was repeatedly washed in an HBSS and was then chopped into small fragments. The fragments were suspended in 12.5 mL of a culture medium prepared by adding 500 U/mL of collagenase type XI (manufactured by Sigma-Aldrich Corporation), 0.4 U/mL of dispase (manufactured by Hoffmann-La Roche Ltd.), and 1 mM dithiothreitol (DTT) to a Dulbecco's modified Eagle's medium (DMEM) containing 1% FBS (complete DMEM). The resulting suspension was shaken at 37°C for 15 minutes to perform an enzymatic reaction. In order to further homogenize undigested fragments, the tissue fragments were allowed to pass through an 18 gauge needle ten times, followed by addition of 10 mL of a complete DMEM thereto. The tube was vigorously shaken and was then left to stand for 1 minute under the force of gravity. The upper portion (10 mL) of the crypt suspension was transferred to another tube. The residual pellet was further allowed to pass through an 18 gauge needle five times, followed by addition of 10 mL of a complete DMEM thereto. This procedure was repeated five times. The suspensions containing free colonic crypts were combined, followed by centrifugation. The precipitate prepared by the centrifugation was suspended in 10 mL of a 30% percoll (manufactured by GE Healthcare)/HBSS solution, followed by centrifugation at 860 g for 5 minutes. Since the percoll density gradient centrifugation separated most of the cell debris, fats, and fibrous substances into the upper portion of the solution, the colonic crypts could be concentrated. Furthermore, in order to remove bacteria or single cells from the colonic crypts, the colonic crypts were resuspended in 10 mL of a complete DMEM containing 2% D-sorbitol, followed by centrifugation at 300 g for 3 minutes. The precipitate prepared by the centrifugation was allowed to pass through a 70-µm cell strainer (manufactured by BD Biosciences) to remove the coarse substances such as bacteria and simple cells. The purified colonic crypts were centrifuged and were washed with advanced DMEM/F12 (manufactured by Invitrogen). The number of the colonic crypts was counted. The precipitate thus-prepared by centrifugation of the purified colonic crypts was used in culture.

The colonic crypts were cultured by the culture method in accordance with the "TMDU protocol" shown below. Two thousand colonic crypts were suspended in 200 µL of a collagen type I solution (manufactured by Nitta Gelatin Inc.), and the suspension was placed in a 48-well plate. After polymerization of the collagen, 500 µL of advanced DMEM/F12 containing 1% BSA (manufactured by Sigma-Aldrich Corporation), 30 ng/mL of mWnt3a (manufactured by R&D Systems, Inc.), 500 ng/mL of mouse Rspo1 (mRspo1) (manufactured by R&D Systems, Inc.), 20 ng/mL of mEGF (manufactured by Peprotech, Inc.), 50 ng/mL of mHGF (manufactured by R&D Systems, Inc.), and 50 ng/mL of mNoggin (manufactured by R&D Systems, Inc.) (TMDU culture medium) was added to each well. The culture medium was replaced with fresh one every other day. In order to perform subculture, the whole gel was digested at 37°C for 5 minutes in a DMEM containing collagenase type XI, and collected organoids were washed with a PBS containing BSA. The organoid precipitate was suspended in a PBS containing 2 mM EDTA and 0.5% BSA, followed by vigorous shaking. The resulting disaggregated small colonic organoid clusters were mixed with a collagen type I solution. The mixture was used in subculture. The size of a cell cluster that is used in subculture from a single cell to a cell cluster consisting of a plurality of cells was regulated by the EDTA treatment time under observation with a microscope. During the first two days after the cell proliferation, 10 µM Rho kinase inhibitor, Y-27632, was added. In the case of inducing differentiation of goblet cells, the colonic organoids were treated with a γ-secretase inhibitor, LY-411,575 (100 nM), for a predetermined period of time.

### [Chromosome analysis]

Chromosome karyotype was analyzed using a Carnoy's fixative solution. Cells were collected from colonic organoids by the same procedure as in the subculture. After centrifugation, the cells were resuspended in a 75 mM KCl solution and were incubated at 37°C for 15 minutes, followed by centrifugation. The cells were resuspended in a methanol-acetic acid (3 : 1) solution to fix the cells. The methanol-acetic acid (3 : 1) solution containing the fixed cells was dropped onto a slide glass, followed by air drying. The chromosomes were stained with DAPI and were observed with a microscope. The number of chromosomes in metaphase was counted for ten cells in total.

### [Histological analysis]

Images of isolated colonic crypts, colonic organoids during growth, and the whole colon tissue of a recipient mouse were taken with a PlanApo 4 × 0.2 NA or PlanApo 20 × 0.75 NA objective lens (manufactured by Nikon Corporation) and a fluorescence microscope including a phase difference setting part (BZ-8000, manufactured by KEYENCE Corporation). Some images of epithelium transplanted in damaged colon tissue were taken with a fluorescence stereoscopic microscope system MVX10 (manufactured by Olympus Corporation). Fluorescent images of colorectal epithelium sections were taken with a fluorescence microscope IX-71 (manufactured by Olympus Corporation) having a UplansApo 10 × 0.4 NA or UplansApo 20 × 0.75 NA objective lens (manufactured by Olympus Corporation) and an Delta Vision system (manufactured by Applied Precision Ltd.). Frozen sections for histological and immunohistochemical analysis were produced by fixing tissue or colonic organoids in collagen gel with 4% paraformaldehyde at 4°C overnight, then successively dehydrating the tissue or colonic organoids in PBSs having different sucrose concentrations of 10%, 15%, and 20%, and freezing the tissue or colonic organoids in a frozen tissue-embedding medium (OCT Compound, manufactured by Tissue Tek). Six-micrometer-thick frozen sections were analyzed by conventional methods: H&E staining, alcian blue staining, and immunohistochemical analysis.

### [Transmission electron microscope]

Collagen type I gel containing colonic organoids during proliferation was fixed with a 0.1 M PBS containing 2.5% glutaraldehyde for 2 hours, then washed in a 0.1 M PBS buffer at 4°C overnight, post-fixed with a 0.1 M PBS containing 1% O_{S}O₄ for 2 hours, then dehydrated with serially diluted ethanol, and embedded in Epon 812. Ultrathin sections (90 nm) were collected on a copper grid and were double stained with uranyl acetate and lead citrate. The sections were analyzed with a transmission electron microscope (H-7100, manufactured by Hitachi, Ltd.).

### [Real-time imaging]

Real-time imaging was performed with a DeltaVision system. A culture dish having a glass bottom was placed on a microscope stage and was covered with a chamber into which premixed humidifier gas composed of 5% CO₂ and 95% air continuously infused. Furthermore, the whole was covered with a temperature-controlling chamber set at 37°C. As real-time images, differential interference contrast (DIC) images and fluorescent images were taken at 20-minute intervals. These image data were processed with softWorx (manufactured by Applied Precision Ltd.), and image editing was optionally performed with Adobe (registered trademark) Photoshop (registered trademark) Elements 7.0 (manufactured by Adobe).

### [Semi-quantitative RT-PCR]

Total RNA was isolated with a TRIzol reagent (manufactured by Invitrogen), and cDNA was synthesized using 1 µg of the total RNA in a 21-µL reaction system. DNA was amplified by PCR using 1 µL of the cDNA in a 25-µL reaction system. The primer sequences used in the PCR and the conditions for the semi-quantitative PCR are shown in Table 1 below. The PCR products were separated on agarose gel, stained with ethidium bromide, and visualized with an ImageQuant TL system (manufactured by GE Healthcare).

**[Table 1]**

| Gene | Forward primer (5'-3') | Reverse primer (5'-3') | Annealing temperature (°C) | Cycle number |
|---|---|---|---|---|
| Gapdh | CTGGCCAAGGTCATCCATGA (SEQ ID NO: 1) | GCCATGAGGTCCACCACCCTG (SEQ ID NO: 2) | 60 | 19 |
| Muc2 | CCTTAGCCAAGGGCTCGGAA (SEQ ID NO: 3) | GGCCCGAGAGTAGACCTTGG (SEQ ID NO: 4) | 60 | 25 |
| CgA | CTGTCAGCCCTGAGTGTCTG (SEQ ID NO: 5) | ATGGAAGTGGGAACTGGATG (SEQ ID NO: 6) | 58 | 31 |
| CAII | AGCACAACGGACCAGAGAAC (SEQ ID NO: 7) | CTGACAGTAATGGGCTCCCT (SEQ ID NO: 8) | 60 | 22 |
| Cdh1 | TGGACAGAGAAGACGCTGAG (SEQ ID NO: 9) | ATCATCATCTGGTGGCAGCA (SEQ ID NO: 10) | 63 | 25 |
| Lgr5 | CTGACTTTGAATGGTGCCTCG (SEQ ID NO: 11) | ATGTCCACTACCGCGATTAC (SEQ ID NO: 12) | 58 | 30 |
| α-SMA | CGCTGTCAGGAACCCTGAGA (SEQ ID NO: 13) | ATGAGGTAGTCGGTGAGATC (SEQ ID NO: 14) | 63 | 25 |

### [Sorting of single Lgr5⁺ cells and culture by Hubrecht protocol (hereinafter, referred to as "HI protocol")]

An R26R-Confetti mouse mated with an Lgr5-EGFP-IRES-CreERT2 mouse was injected with 5 mg of tamoxifen, and the colonic crypts were isolated 3 days after the cre induction. The epithelial cells were dissociated with TrypLE express (manufactured by Invitrogen) at 37°C for 2 hours, were allowed to pass through a 20-µm cell strainer (manufactured by Celltrix), were washed with a PBS, and were analyzed with MoFlo (manufactured by DakoCytomation Inc.). The cell populations were gated by forward scattering, side-way scattering, and a pulse width parameter, and dead cells were removed by negative staining with propidium iodide or 7-ADD (manufactured by eBioscience). EGFP/RFP-double-positive cells were sorted, were embedded in matrigel (manufactured by BD Biosciences), and were seeded in a 96-well plate. Advanced DMEM/F12 containing penicillin, streptomycin, 10 mM HEPES, Glutamax, 1 × N2, and 1 × B27 (all of them manufactured by Invitrogen) and growth factors (50 ng/mL of EGF, 100 ng/mL of noggin, and 1 µg/mL of R-spondin) was diluted with a Wnt3a conditioned culture solution at 1 : 1, and the resulting medium was used as a colonic crypt culture medium (Hubrecht culture medium). In order to prevent anoikis, a ROCK inhibitor, Y-27632, (10 µM) was added to the culture medium for the first two days. The growth factors were added to the culture medium every other day, and the entire culture medium was replaced with fresh one every four days.

### [Experimental transplantation]

Cells isolated from colon tissue were cultured for 5 to 8 days in accordance with the culture method in the TMDU protocol and were used as EGFP⁺ cells for transplantation. Sorted EGFP⁺/RFP⁺ cells were propagated for 5 to 10 weeks in accordance with the HI protocol and were then cryopreserved in Recovery™ Cell Culture Freezing Medium (manufactured by GIBCO) as colonic organoids derived from single Lgr5⁺ cells for transplantation. When the cells were thawed and then used, the cells were cultured in accordance with the HI (Hubrecht) protocol for at least 5 weeks and then in accordance with the TMDU protocol at least 1 week before the transplantation. Acute colitis was induced by feeding RAG2^{-/-} mice for 5 days (on 1st to 5th days) with water dissolving 3.0% DSS (molecular weight: 10,000, manufactured by Ensuiko Sugar Refining Co.). The severity of colitis was evaluated by measuring the body weights of the mice. The donor colonic organoids were collected from the collagen type I gel on the 7th and 10th days from the start of administration of DSS, and the intercellular adhesion was dissolved with EDTA, followed by washing with a PBS containing BSA as in the procedure of subculture. Cell clusters in an amount corresponding to that obtained from about 500 organoids were resuspended in 200 µL of matrigel diluted with a PBS (1 : 20). The cell suspension was injected into the colonic lumen with a syringe and a thin flexible catheter having a length of 4 cm and a diameter of 2 mm. In order to prevent that the lumenal contents are excreted immediately after the injection, the anal verge was bonded for 6 hours. The mice were commonly maintained after the transplantation on the 10th day until slaughtered for analysis.

### [Tetramethylrhodamine isothiocyanate (TRITC)-dextran permeation assay]

A nonmetabolizable macromolecule, TRITC-dextran (molecular weight: 4.4 kDa, manufactured by Sigma-Aldrich Corporation), used as a permeable probe was administered to evaluate intestinal permeability. TRITC-dextran (4 mg/10 g body weight) was orally administered into the gastrointestinal tract of the transplantation mouse and sham transplantation mouse with a tube 4 hours before slaughter. Exsanguination was performed by cardiopuncture at the time of the slaughter, and the colon tissue was investigated for whether the EGFP⁺ graft was present or not. The TRITC-dextran was measured twice for each sample with ARVO MX (manufactured by PerkinElmer Inc.) using a standard curve drawn from serial dilution solutions of a PBS solution containing TRITC-dextran.

### [Method of isolating, culturing, or subculturing human colorectal epithelial stem cells]

### (1) Method of isolating human colorectal epithelial stem cells

1) Human colon tissue of about 5 to 6 mm diameter was collected during colonoscopy with a forceps that is usually used in examination of tissue.
2) The human colon tissue was immediately transferred into an HBSS containing antibiotics (hereinafter abbreviated to Ab: 100 U/mL of penicillin, 100 mg/mL of streptomycin, and 50 mg/mL of gentamycin) in a tube cooled at 4°C on ice in advance.
3) The tube was transported from the endoscopic room to a laboratory without delay with maintaining the low temperature of 4°C.
4) The human colon tissue was washed with an HBSS containing Ab twice by shaking the tube.
5) The human colon tissue was transferred onto a petri dish and was chopped with a razor blade while the HBSS containing Ab was being removed.
6) The chopped human colon tissue was suspended in 12.5 mL of a culture medium prepared by adding 500 U/mL of collagenase type XI (manufactured by Sigma-Aldrich Corporation), 0.4 U/mL of dispase (manufactured by Hoffmann-La Roche Ltd.), and 1 mM dithiothreitol (DTT) to a Dulbecco's modified Eagle's medium (DMEM) containing 1% FBS (complete DMEM), and the suspension was transferred into a 50-mL Falcon tube (manufactured by Becton, Dickinson and Company).
7) Digestion treatment was performed at 37°C with shaking for 7 minutes.
8) After the digestion treatment, chopping with a 10-mL syringe with an 8G needle was further performed ten times.
9) After addition of 20 mL of a serum-free DMEM containing Ab and 2% D-sorbitol, centrifugation was performed at 1180 rpm for 3 minutes.
10) The resulting precipitate pellet was suspended in 10 mL of 30% percoll (manufactured by GE Healthcare)/HBSS solution, and the suspension was transferred to a 15-mL tube, followed by centrifugation at 2000 rpm for 5 minutes.
11) The resulting precipitate pellet was suspended in 10 mL of a serum-free DMEM containing Ab and 2% D-sorbitol, and the suspension was allowed to pass through a 300-µm mesh to remove coarse tissue and was collected in a 15-mL stemfull tube (manufactured by Sumitomo Bakelite Co., Ltd.).
12) A serum-free DMEM (10 mL) containing Ab was added to the tube, followed by centrifugal washing at 1500 rpm for 3 minutes twice.
13) The number of resulting colonic crypts was counted.

### (2) Method of culturing human colorectal epithelial stem cells

1) The colonic crypts were mixed with a collagen type I solution (manufactured by Nitta Gelatin Inc.) such that an appropriate amount of the colonic crypts were dispensed in each well of a culture (24-well) plate.
2) The mixture solution (50 µL) of the cells and the collagen type I solution was dropped into each well such that the mixture solution was formed into a dome-like shape at the center of the culture well.
3) After confirmation of gelation of the collagen by leaving to stand at 37°C for 30 minutes, 500 µL of a culture medium (shown below) was added to the well.
4) The culture medium replacement was performed every 3 days.

### (3) Method of subculturing human colorectal epithelial stem cells

1) Subculture was performed every 6 to 10 days at the time when colonic organoids became large.
2) The culture medium surrounding the cells was completely sucked in such a manner that the gel containing the cells was not destroyed.
3) The gel was peeled from the culture dish by sucking the gel with a 2-mL pipette and was collected in a 15-mL stemfull tube.
4) A serum-free DMEM containing 500 U/mL of collagenase type XI (manufactured by Sigma-Aldrich Corporation, c4785, 130 µL/1000 µL) was added to the tube.
5) The collagen gel was digested by shaking at 37°C for 5 minutes.
6) After washing with 10 mL of an HBSS once, centrifugation was performed at 2000 rpm for 3 minutes.
7) Three milliliter of Trypsin/EDTA (manufactured by GIBCO) was added thereto, followed by shaking at 37°C for 5 minutes.
8) Shaking was repeated three to five times while observing with a microscope until the colonic organoids were dispersed in cell clusters.
9) After washing with 10 mL of a serum-free DMEM twice, centrifugation was performed at 2000 rpm for 3 minutes.
10) The precipitate was mixed with a collagen type I-A solution (50 µL per well), and 45 µL of the mixture was seeded in a coated 24-well culture dish such that the mixture was formed into a dome-like shape at the center of the culture well.
11) After confirmation of gelation of the collagen by culturing at 37°C for 30 minutes, 500 µL of a culture solution was added to the culture dish.
12) The culture medium replacement was performed every 3 days.

### (4) Culture medium

In the method of isolating, culturing, or subculturing human colorectal epithelial stem cells, a culture medium having, for example, a composition shown in Table 2 below was used.

**[Table 2]**

| | Final concentration | Culture medium |
|---|---|---|
| Ad DMEM/F 12 (Gibco) | | 1 ml |
| Penicillin-Streptomycin (Nacalai) | 50 U/ml 50 ng/ml | 5 µl/1 ml |
| Gentamycin (Nacalai) 10 mg/ml | 25 µg/ml | 2.5 µl/1 ml |
| L-Glutamin (Gibco) 200 mM | 2 mM | 10 µl/1 ml |
| Bovine Serum Albumin (Sigma) 30% | 1% | 33 µl/ml |
| Mouse Noggin (R&D) 20 µg/ml | 50 ng/ml | 2.5 µl/ml |
| Mouse R-Spondinl (R&D) 100 µg/ml | 500 ng/ml | 5 µl/ml |
| Mouse EGF (Peprotech) 20 µg/ml | 20 ng/ml | 1 µl/ml |
| Human HGF (R&D) 100 µg/ml | 50 ng/ml | 0.5 µl/ml |
| mouse Wnt3a (R&D) 10 µg/ml | 300 ng/ml | 30 µl/ml |
| Y-27632 (Calbiochem) 5 mM | 10 µM | 2 µl |

### [Statistical analysis]

Experimental data are expressed as means ± SEM. The statistical analysis was performed by comparing the results of each group by a paired student T test or a Mann-Whitney U test. The statistical significance in the comparison was defined as P < 0.05.

### [Results]

Colonic crypts substantially not containing non-epithelial tissue components could be isolated from the colon tissue of an adult mouse using an enzyme (Booth, C., Patel, S., Bennion, G.R. & Potten, C.S., The isolation and culture of adult mouse colonic epithelium, Epithelial Cell Biol., 4, 76-86 (1995)), a reducing agent (Whitehead, R.H., Demmler, K., Rockman, S.P. & Watson, N.K., Clonogenic growth of epithelial cells from normal colonic mucosa from both mice and humans, Gastroenterology, 117, 858-865 (1999)), and mechanical homogenization. Gene expression analysis of the colonic crypts revealed that E-cadherin-positive epithelial cells including both terminal differentiation marker genes (MUC2, CAII, and CgA) expressing cells and Lgr5⁺ cells were collected and that non-epithelial cells such as α-SMA-positive myofibroblasts were efficiently removed.

Next, methods of culturing colonic crypt cells were investigated. It is known that in order to culture small intestinal cells, three factors, Rspo1 (R-spondin 1), Noggin, and EGF, are indispensable (Sato, T., et al., Single Lgr5 stem cells build crypt-villus structures in vitro without a mesenchymal niche, Nature, 459, 262-265 (2009)). Accordingly, culture of colonic crypt cells was tried using these three factors, but culture of colonic crypt cells could not be maintained. Accordingly, the culture method was modified by adding the following matters 1) to 4) to the method of culturing small intestinal cells: 1) colonic crypt cells are three-dimensionally embedded in collagen type I gel and are cultured in a serum-free culture medium; 2) Wnt signaling pathway is activated, and Wnt3a (Barker, N., et al., Lgr5(+ve) stem cells drive self-renewal in the stomach and build long-lived gastric units in vitro, Cell Stem Cell, 6, 25-36 (2010)) indispensable for in vitro propagation of gastric epithelium is used; 3) hepatocyte growth factor (HGF), which is known to be a pleiotropism factor derived from mesenchyme regulating proliferation of various cell types such as colorectal epithelial cells (Kanayama, M., et al., Hepatocyte growth factor promotes colonic epithelial regeneration via Akt signaling, Am. J. Physiol. Gastrointest. Liver Physiol., 293, G230-239 (2007), Tahara, Y., et al., Hepatocyte growth factor facilitates colonic mucosal repair in experimental ulcerative colitis in rats, J. Pharmacol. Exp. Ther., 307, 146-151 (2003)), is used; and 4) bovine serum albumin (BSA), which is one of components of a B27 supplement necessary for long-time cell culture (Barker, N., et al., Lgr5(+ve) stem cells drive self-renewal in the stomach and build long-lived gastric units in vitro, Cell Stem Cell, 6, 25-36 (2010)) is used. That is, it was revealed that colonic crypt cells are healthily propagated by culturing the colonic crypt cells (the culture method in the "TMDU protocol" of the culture method disclosed herein)
using a culture medium containing mWnt3a, mHGF, and BSA, in addition to the three factors (mRspo1, mNoggin, and mEGF) used in culture of small intestinal cells.

In addition, observation of the manner of proliferation of colonic crypt cells by the culture method in the "TMDU protocol" revealed that the colonic crypt cells rapidly proliferate and form a spherical cyst structure having a closed lumenal space to form an organoid (Figure 1a). Whether Wnt3a, Rspo1, and BSA are necessary for maintaining the culture of colonic crypt cells was investigated, and it was revealed that the culture of colonic crypt cells cannot be maintained when any one of these components is absent (Figure 2a). This result demonstrates that three factors, Wnt3a, Rspo1, and BSA, are indispensable for maintaining the culture of colonic crypt cells. On the other hand, though three factors, Noggin, EGF, and HGF, were not essential, each of them enhanced the proliferation of colonic crypt cells (Figure 2b).

Next, the colonic organoid formed by the culture method disclosed herein was analyzed in detail with cellular level. The colonic organoid was composed of monolayer cells and was positive in staining using an antibody against E-cadherin, which revealed that the colonic organoids of the present invention have histological characteristics of epithelial cells (Figure 1b, the first one from the left). Meanwhile, the basal side of the colonic organoid always faced the outside (Figure 1b, the leftmost one). In addition, a large number of cells detected by anti-Ki67 antibody staining, which is an indicator of growing cells, were observed (Figure 1b, the second one from the left). This result demonstrates that actively proliferating cells are abundantly present in the colonic organoid of the present invention. In order to investigate whether terminally differentiating cells are present in the colonic organoids, staining using alcian blue (staining goblet cells), an anti-CgA antibody (detecting enteroendocrine cells), an anti-CAII antibody (detecting mature colonocytes), and an anti-Coxl antibody (detecting tuft cells (Gerbe, F., et al., Distinct ATOH1 and Neurog3 requirements define tuft cells as a new secretory cell type in the intestinal epithelium, J. Cell. Biol., 192, 767-780 (2011)) was performed. It was revealed that there existed cells detected by alcian blue staining (Figure 1b, the third one from the left), the anti-CgA antibody (Figure 1b, the third one from the right), the anti-CAII antibody (Figure 1b, the second one from the right), and the anti-Coxl antibody (Figure 1b, the first one from the right). The results demonstrate that terminally differentiated colonocytes were present in the colonic organoid of the present invention. Furthermore, analysis of the colonic organoid with a transmission electron microscope revealed that the colonic organoid has characteristics of epithelial cells, such as microvilli (Figure 1c) and intercellular junctional complex (Figure 1d), and that these cells are arranged in a monolayer to surround the lumen. Actively proliferating cells containing condensed chromosomes characteristic to mitotic cells were present (Figure 1e). In addition, terminally differentiated cells such as goblet cells containing translucent secretory vesicles (Figure 1f) and enteroendocrine cells containing small electron-dense granules (Figure 1g) were present. The results shown in Figures 1e to 1g support the results of immunohistochemical staining shown in Figure 1b.

Next, it was investigated whether colonic crypt cells obtained from destroying the organoid reconstruct an organoid structure. The colonic organoids were destroyed into cell clusters or single cells. The resulting cells were embedded in fresh collagen gel at a ratio of 1 : 2 (cells : collagen) and were cultured. As a result, an organoid having a cyst structure was reconstructed. As shown in subculture of small intestinal cells (Sato, T., et al., Single Lgr5 stem cells build crypt-villus structures in vitro without a mesenchymal niche, Nature, 459, 262-265 (2009)), the replating efficiency of colonocytes was increased by a Rho kinase inhibitor Y-27632 (Watanabe, K., et al., A ROCK inhibitor permits survival of dissociated human embryonic stem cells, Nat. Biotechnol., 25, 681-686 (2007)). Furthermore, even when the culture of the cells was continued over 6 months or more in total with subculture once a week using the Rho kinase inhibitor Y-27632, the cells could propagate a colonic organoid without changing the characteristics (Figure 1h) and karyotype (Figure 1i).

Next, maintaining of the stem cells of a colonic organoid was investigated. The cells were collected on the 1st, 8th, and 60th days of subculture, and the expression of the mRNA of Lgr5, which is a colorectal epithelial stem cell marker, was analyzed. As a result, the expression of Lgr5 mRNA was detected at least for 60 days of the culture and was significantly enhanced, in particular, on and after the 8th day (Figure 3a). Though the expression levels of the CgA gene and the CAII gene did not change immediately after the culture of the colonic crypt cells, the expression level of the MUC2 gene, which is known as a goblet cell marker gene, was suppressed by the culture of the colonic crypt cells (Figure 3a). The expression of the Lgr5 gene was induced by a combination of Wnt3a, Rspo1, and BSA. This result demonstrates that Wnt signaling is important for maintaining the sternness (Figure 3b). Furthermore, the expression of the Lgr5 gene was further enhanced by Noggin, which is an antagonist of BMP signaling (Haramis, A.P., et al., De novo crypt formation and juvenile polyposis on BMP inhibition in mouse intestine, Science, 303, 1684-1686 (2004)) (Figure 3b). This result demonstrates that BMP signaling is antagonistic to maintenance of sternness. It is also known that the Notch pathway is involved in cell fate determination by suppressing differentiation to secretion series of precursor cells (Fre, S., et al., Notch signals control the fate of immature progenitor cells in the intestine, Nature, 435, 964-968 (2005), van Es, J.H., et al., Notch/gamma-secretase inhibition turns proliferative cells in intestinal crypts and adenomas into goblet cells, Nature, 435, 959-963 (2005)) and stem cells (van Es, J.H., de Geest, N., vande Born, M., Clevers, H. & Hassan, B.A., Intestinal stem cells lacking the Math1 tumour suppressor are refractory to Notch inhibitors, Nat. Commun., 1, 1-5 (2010)). Accordingly, it was investigated whether the Notch pathway is involved in the maintenance of sternness of the cultured colonic organoid. It was recognized that treatment of the colonic organoid of the present invention with a γ-secretase inhibitor (GSI), LY-411,575, inhibiting the Notch signaling (Wong, G.T., et al., Chronic treatment with the gamma-secretase inhibitor LY-411,575 inhibits beta-amyloid peptide production and alters lymphopoiesis and intestinal cell differentiation, J. Biol. Chem., 279, 12876-12882 (2004), Okamoto, R., et al., Requirement of Notch activation during regeneration of the intestinal epithelia, Am. J. Physiol. Gastrointest. Liver Physiol., 296, G23-35 (2009)) differentiated most of the cells to goblet cells (Figure 3c, the left), enhanced the expression of the MUC2 gene, and, in contrast, decreased the expression of the Lgr5 gene (Figure 3c, the right). This result demonstrates that the Notch signal is involved in maintenance of sternness of the colonic organoids of the present invention.

Next, it was investigated whether the increase in Lgr5 mRNA level recognized in the colonic organoid of the present invention is caused by an increase in the number of stem cells or by an increase in the Lgr5 gene expression level in each stem cell. First, colonic crypt cells were isolated from an Lgr5-EGFP-ires-CreERT2 mouse in which an EGFP- and tamoxifen-inducible Cre recombinase cassette has been incorporated into the Lgr5 locus (Barker, N., et al., Identification of stem cells in small intestine and colon by marker gene Lgr5, Nature, 449, 1003-1007 (2007)), and the manner that the colonic crypt cells form a colonic organoid was analyzed by real-time imaging. The expression of EGFP induced by an Lgr5 promoter (hereinafter, simply referred to as "expression of EGFP" or "signal of EGFP") was detected by means of fluorescence of EGFP. The expression of EGFP was slightly observed in a restricted region of the colonic crypts immediately after the isolation of the colonic crypt cells (0 h on the 1st day), but the expression of EGFP was not substantially detected for the subsequent several days. The signal of EGFP was observed from around the 5th day (128 h), and strong signals of EGFP were observed at multiple regions of the colonic organoid over the subsequent several days (Figure 3d). This result demonstrates that cells expressing EGFP, i.e., Lgr5⁺ cells, have proliferated. For detailed analysis with a resolution that allows detection of an individual cell, organoid serial sections were produced on the 6th day of the culture of the colonic crypt cells, the signal of EGFP in each section was detected. As a result, a plurality of EGFP⁺ cells were observed at a plurality of regions, and it was revealed that these cells occupy 40% to 50% of the cells of the whole structure. This result supports the result of the real-time imaging described above that the Lgr5⁺ cells have proliferated. An important point is that the number of EGFP⁺ cells in the individual organoid highly varies. This result suggests a tendency of suppression (silencing) of Lgr5 promoter-EGFP locus during the subculture over a large number of generations. On the other hand, the expression of a wild-type (wt) promoter-Lgr5 allele was maintained at a certain level (Figures 3a and 3b). A similar phenomenon was also observed in small intestinal organoids. The results above demonstrate that colon Lgr5⁺ stem cells can proliferate in vitro by self-renewing as in small intestine Lgr5⁺ stem cells (Sato, T., et al., Single Lgr5 stem cells build crypt-villus structures in vitro without a mesenchymal niche, Nature, 459, 262-265 (2009)).

Next, it was investigated whether stem cells derived from the colonic organoids of the present invention cultured in vitro (hereinafter, simply referred to as "colorectal epithelial stem cells of the present invention") can regenerate colorectal epithelium in vivo. First, a mouse model having colitis, which is similar to inflammatory bowel disease (IBD) in human, was produced (Wirtz, S., Neufert, C., Weigmann, B. & Neurath, M.F., Chemically induced mouse models of intestinal inflammation, Nat. Protoc. 2, 541-546 (2007)). Specifically, chemical-induced inflammation was induced in the colonic mucous membrane by feeding an RAG2^{-/-} mouse with impaired immune function with water containing dextran sulfate sodium (DSS) for 5 days. It was observed that the mouse developed acute colitis characterized by a decrease in body weight, hematochezia, and diarrhea, in particular, significantly damaged epithelium at the farthest portion of the colon, i.e., near the anus. Subsequently, in order to transplant the colorectal epithelial stem cells of the present invention to the colorectal epithelium by enema, a simple and specific approach of transporting the stem cells to the colitis tissue has been planned, and a transplantation strategy as an embodiment thereof has been invented (Figure 4a). Herein, in order to differentiate a colonic crypt derived from a colitis model mouse and a graft (colorectal epithelial stem cells of the present invention), EGFP-expressing transgenic mice (Okabe, M., Ikawa, M., Kominami, K., Nakanishi, T. & Nishimune, Y., 'Green mice' as a source of ubiquitous green cells, FEBS Lett., 407, 313-319 (1997)) were used as donor mice. Colonic organoids derived from each EGFP⁺ donor mice were dissociated into small fragments on the 7th and 10th days from the start of DSS administration to RAG2^{-/-} mice. The colorectal epithelial stem cells of the present invention were suspended in a PBS containing matrigel, and the suspension was injected into the colon of the colitis model mice (hereinafter, also referred to as "recipient" or "recipient mouse").

On the 6th day from the second transplantation (on the 16th day from the start of DSS administration), the recipient mice transplanted with the colorectal epithelial stem cells of the present invention showed a decrease in the damage level of the colorectal epithelium compared to the recipient mice with sham transplantation, but still had mucous membrane loss surrounded by edematous regions of the distal colon (Figure 4b). Interestingly, in the recipient of transplantation, a plurality of EGFP⁺ (indicating colorectal epithelial stem cells of the present invention) regions was observed as clear boundaries mainly in the recessed lesion site (Figure 4b). This result suggests that the transplanted colorectal epithelial stem cells of the present invention adhered to the damaged mucous membrane. As the proof thereof, in RAG2^{-/-} mice to which DSS was not administered, such an EGFP⁺ region was not observed at any site of the colon. Accordingly, whether the transplanted colorectal epithelial stem cells of the present invention adhered to the damaged mucous membrane of the recipient was histologically analyzed in detail. The results demonstrate that EGFP⁺ cells covered the damaged mucous membrane with colonic epithelial loss among recipient tissues (lower panel in Figure 4c, arrowhead) maintaining the crypt structure with a low degree of damage. In addition, the EGFP⁺ cells formed a flat sheet-like arrangement (upper panel in Figure 4c, outlined narrow arrow) or slightly recessed structure resembling the colonic crypt (upper panel in Figure 4c, outlined wide arrow).

Furthermore, it was observed that some of EGFP⁺ cells bound to the recipient epithelium at a distal end of the damaged site (Figure 4d, outlined arrowhead and white arrowhead) and that some of EGFP⁺ cells adhered to a region where no recipient colorectal epithelium was present (Figure 4e, outlined arrowhead and white arrowhead). These results demonstrate that exogenously supplied colorectal epithelial stem cells of the present invention can compensate colonic epithelial damage of a recipient by various patterns. It was also demonstrated that the recipient mice transplanted with the colorectal epithelial stem cells of the present invention recovered the body weight earlier than the mice with sham transplantation (Figure 4f, on the 12th, 13th, and 14th days, p < 0.05). This result demonstrates that the colorectal epithelial stem cells of the present invention rescue the colonic epithelial damage and also enhance recovery from acute colitis.

Next, the colorectal epithelial stem cells of the present invention were transplanted into a recipient, and the colorectal epithelium in the 4th week was analyzed. As a result, a local region composed of tubular EGFP⁺ crypts was observed (Figure 5a). This result demonstrates that EGFP⁺ cells derived from the colorectal epithelial stem cells of the present invention were engrafted in the damaged epithelium region of the distal terminal of the colon and survived over 4 weeks. The EGFP⁺ cells were histologically demonstrated to form monolayer epithelium having a mature crypt structure with a deep invagination, as in the surrounding cells (EGFP⁻ cells) derived from the recipient (Figure 5b, the left). A particularly notable point is that in a crypt containing EGFP⁺ cells, all cells in the crypt are EGFP⁺ cells. These results suggest that the colorectal epithelial stem cells cultured in vitro of the present invention function as colorectal epithelial stem cells even in the colorectal epithelium returned to the body by transplantation (Figure 5b, the right). The results of immunohistochemical staining were that the cells localized at the bottom of EGFP⁺ crypts and positive to anti-Ki67 antibody staining show the same distribution as that of the cells of EGFP⁻ crypts derived from the recipient, in the vicinity thereof, and positive to anti-Ki67 antibody staining. The results thus revealed that the cells in the graft actively proliferated (Figure 5c, the first one from the left). Furthermore, analysis of serial sections of the tissue revealed that the EGFP⁺ crypts derived from a donor include every terminally differentiated cells, i.e., goblet cells (Figure 5c, the second one from the left), enteroendocrine cells (Figure 5c, the third one from the left), mature colonocytes (Figure 5c, the second one from the right), and tuft cells (Figure 5c, the first one from the right) . These results demonstrate that in the recipient mouse, the transplanted colorectal epithelial stem cells of the present invention were engrafted at the region where the tissue-resident stem cells were lost and replaced the loss. Furthermore interestingly, it was demonstrated that the transplantation efficiency (evaluated by the area of EGFP⁺ cell region) was significantly high in the case of transplanting colorectal epithelial cells propagated by the method of culturing colorectal epithelial stem cells disclosed herein compared to the case of transplanting colorectal epithelial cells immediately after isolation from the colon tissue to a recipient mouse (p < 0.05, Mann-Whitney U test). This result suggests that the transplantation efficiency is increased by using colorectal epithelial stem cells capable of propagating stem cells in vitro of the present invention. Furthermore, the transplantation efficiency was increased in the recipient mouse transplanted with a colonic organoid suspension containing matrigel compared to that in the recipient mouse transplanted with colonic organoids suspended in a PBS (p < 0.05, Mann-Whitney U test). This result suggests that transplantation of an extracellular matrix component such as matrigel together with colorectal epithelial stem cells of the present invention causes some effects, such as adhesion of the colorectal epithelial stem cells of the present invention to a damaged site and enhancement of prompt engrafting during the transplantation process, thereby increasing the transplantation efficiency.

In order to investigate whether the colorectal epithelium transplanted and engrafted with the colorectal epithelial stem cells of the present invention has epithelial integrity having a normal barrier function, a probe for an epithelial permeability test, tetramethylrhodamine isothiocyanate (TRITC)-conjugated dextran (TRITC-dextran), was enterally administered to a transplantation mouse group (DSS(+), engraft(+)) transplanted with colorectal epithelial stem cells of the present invention and a control mouse group (DSS(+), sham) received sham transplantation, and the transmucosal transportation of the TRITC-dextran was investigated. This experiment utilizes properties that dextran having a large molecular weight is not absorbed from normal intestinal epithelium, but if the intestinal epithelium has abnormality such as inflammation, dextran is absorbed from the gap between cells to increase the blood TRITC-dextran level (blood TRITC level). The right graph in Figure 5d shows blood TRITC levels in DSS administration mice (DSS(+)) on the 6th day from the start of administration of DSS for successive 5 days and blood TRITC levels in control mice (DSS(-)) not administered with DSS. The graph shows that the blood TRITC level was significantly increased in DSS(+) compared to DSS(-). This demonstrates that this experiment system can discriminate the state of the barrier function of intestinal epithelium.

On the other hand, the left graph in Figure 5d shows blood TRITC levels in a transplantation mouse group (DSS(+), engraft(+)) transplanted with the colorectal epithelial stem cells of the present invention and blood TRITC levels in a control mouse group (DSS(+), sham) received sham transplantation. The blood TRITC levels were measured on the 38th day from the start of DSS administration, at the time when 4 weeks had already passed from the start of transplantation or sham transplantation. The time when 4 weeks passed from the transplantation corresponds to the time by which colorectal epithelium should have been regenerated by endogenous colorectal epithelial stem cells in the control mouse group (DSS(+), sham) received sham transplantation. The results shown in the left graph in Figure 5d revealed that there was no significant difference between the blood TRITC levels in the transplantation mouse group (DSS(+), engraft(+)) transplanted with the colorectal epithelial stem cells of the present invention and the blood TRITC levels in the control mouse group (DSS(+), sham) received sham transplantation. This demonstrates that the colonic mucous membrane transplanted with the colorectal epithelial stem cells of the present invention maintains the epithelial integrity that is important for the epithelial barrier function that does not transmit large molecules such as dextran. That is, these results demonstrate that extraneous colon stem cells of the present invention can proliferate in vivo and also can have epithelial integrity having a normal barrier function, like endogenous colorectal epithelial stem cells. Even if colorectal epithelial stem cells are transplanted, all of them are not necessarily engrafted. Accordingly, in the transplantation mouse group (DSS(+), engraft(+)) transplanted with the colorectal epithelial stem cells of the present invention, the colon of each mouse was observed by laparotomy, and only individuals in which EGFP-positive grafts derived from the colorectal epithelial stem cells of the present invention were observed were selected and were used for statistical processing of blood TRITC levels in the left graph in Figure 5d.

Next, an experiment of producing colonic organoids from single colorectal epithelial stem cells and transplanting the organoids into recipient mice was performed (Figure 6a). In this experiment, first, in order to trace and visualize descendant cells derived from single colorectal epithelial stem cells, an Lgr5-EGFP-Ires-CreERT2 mouse was mated with an R26R-Confetti reporter mouse (Snippert, H.J., et al., Intestinal crypt homeostasis results from neutral competition between symmetrically dividing Lgr5 stem cells, Cell, 143, 134-144 (2010)), and a recombination reaction by tamoxifen-induced Cre recombinase was induced into the resulting descendant mice. As a result, any of four fluorescent proteins (RFP, CFP, GFP, and YFP) was expressed in the Lgr5⁺ stem cells, and the expression of the fluorescent protein was maintained in all the descendant cells thereof. RFP-expressing Lgr5⁺ stem cells were isolated from the Lgr5⁺ stem cells after the recombination reaction. Specifically, the colonic crypts were isolated from the mice 3 days after the recombination reaction by the Cre recombinase and were prepared into single cells, followed by sorting Lgr5-EGFP⁺/Confetti-RFP⁺ stem cells showing EGFP fluorescence (derived from Lgr5 knockin allele) and RFP fluorescence (derived from R26R-Confetti allele). Such colorectal epithelial stem cells were obtained at a proportion of 100 cells per mouse, which was 0.02% or less of the total number of the cells (Figure 6b).

The colorectal epithelial stem cells obtained by sorting were seeded in each well (100 cells/well of 96-well plate) by limiting dilution so as to give single cells. The colorectal epithelial stem cells were cultured by a "Hubrecht" protocol (a modification method of the method generally known as a small intestinal epithelial stem cell protocol (Sato, T., et al., Single Lgr5 stem cells build crypt-villus structures in vitro without a mesenchymal niche, Nature, 459, 262-265 (2009)) such that single colorectal epithelial stem cells can proliferate) (regarding the details, see above). Under such conditions, four colonic organoids propagated from single colorectal epithelial stem cells were observed. The plating efficiency was equivalent to that observed in small intestinal epithelial stem cells. The cells in these organoids were all RFP⁺, which was reflection of the first recombination at the R26R-Confetti locus before the sorting (Figure 6c). The signal of EGFP (detecting expression level of Lgr5 promoter) was detected several days after the start of the culture (Figure 6c), but became undetectable with time. This result is believed to reflect the silent change of the Lgr5 promoter-EGFP locus as described above. RFP⁺ organoids derived from single colorectal epithelial stem cells exponentially proliferated through serial subculture to at least 100 wells in the 10th week. Subsequently, the organoids were cryopreserved. The cells were thawed and were cultured by the culture method in the "TMDU protocol" before transplantation. The colorectal epithelial stem cells of the present invention prepared from about 500 colonic organoids were injected into the colon of a recipient mouse by the method described above. The results of analysis performed 4 weeks after the transplantation demonstrated again that differentiated cells described above were contained in the colorectal epithelial stem cells of the present invention adhered to the colorectal epithelium of the recipient. In analysis of the recipient mice 17, 21, and 25 weeks after the transplantation, RFP⁺ regions were clearly observed at each time, and it was demonstrated that the grafts derived from the colorectal epithelial stem cells of the present invention were maintained for a long time (Figure 6d shows the results at 25 weeks after the transplantation). Histological analysis of RFP⁺ tissue at 6 months (25 weeks) after the transplantation recognized a monolayer epithelium arrangement of RFP⁺ cells. In addition, no adenomatous change and epithelial dysplasia were observed. This result demonstrates that malignant progression of cells did not occur during the in vitro culture process and during the in vivo tissue formation process after transplantation (Figure 7a). This analysis also demonstrated the presence of terminally differentiated cells and Ki67⁺ growing cells described above in the RFP⁺ crypts (Figure 7b) and no difference in the numbers of alcian blue-positive goblet cells and CgA⁺ enteroendocrine cells between the outside and the inside of the graft. These results further support that colorectal epithelial stem cells derived from single Lgr5⁺ cells and maintaining the multipotency for a long time were present inside the graft and that the colorectal epithelial stem cells constructed colon tissue similar to the recipient epithelium therearound.

Furthermore, whether the method of isolating, culturing, or subculturing the colorectal epithelial stem cells derived from a mouse described above can be applied to human cells was investigated. As a result, it was revealed that colorectal epithelial stem cells can be stably and reproducibly cultured from a minute quantity of colon tissue obtained from a subject during colonoscopy as a starting material, without requiring a large quantity of colon tissue, such as a surgical specimen, as a starting material by employing a [Method of isolating, culturing, or subculturing human colorectal epithelial stem cells] described above.

The colonic crypt cells of a mouse were observed to proliferate in the above TMDU culture medium containing EGF, HGF, and Noggin and further containing another arbitrary component. That is, the colonic crypt cells were cultured by the same procedure as in the "TMDU protocol" described above except that an insulin-transferrin-selenite mixture solution (ITS) (manufactured by Invitrogen) at a 100 times dilution (concentration such that the final concentrations of insulin, transferrin, sodium selenite, and sodium pyruvate were 10 µg/ml, 5.5 µg/ml, 6.7 ng/ml, and 110 µg/ml, respectively), 100 µM (final concentration) of N-acetyl-cysteine (NAC) (manufactured by Nacalai Tesque, Inc.), 200 ng/ml (final concentration) of Cholera Toxin (manufactured by Wako Pure Chemical Industries, Ltd.), a nucleoside mixture solution (manufactured by Sigma-Aldrich Corporation) containing each nucleoside at a concentration of 100 µM (final concentration), and 38 nM (final concentration) of ceruloplasmin (ferroxidase) (manufactured by Sigma-Aldrich Corporation) were further added to a TMDU culture medium. The manner of the proliferation was observed. The results are shown in Figure 8. As obvious from Figure 8, good cell proliferation was observed in the TMDU culture medium further containing ITS, NAC, cholera toxin, and ceruloplasmin. Proliferation of mouse colonic crypt cells was also observed by culturing the cells in the TMDU culture medium containing 10% fetal calf serum (FCS).

### [Conclusion]

The present invention proves that the colorectal epithelial stem cells cultured in vitro of the present invention can be used in stem cell-based therapy for repairing damaged colorectal epithelium. The colorectal epithelial stem cells transplanted in colon tissue having epithelial damage adhere to and cover the site of epithelial loss so as to compensate the lost epithelium. The increase in body weight during the convalescent stage of the recipient of transplantation of the colorectal epithelial stem cells of the present invention is larger than that of the control without transplantation, which demonstrates that the transplantation of the colorectal epithelial stem cells of the present invention reduces the severity of DSS-induced acute colitis. These results demonstrate that the present invention is a promising option for treating a disease having severe gastrointestinal epithelial damage by culturing gastrointestinal epithelial stem cells in vitro while increasing the number of the cells and then transplanting the cells.

### Industrial Applicability

The present invention can be suitably used in the field of in vitro culture of colorectal epithelial stem cells, the field of prevention or treatment of bowel diseases, the field of administration or transplantation of colorectal epithelial stem cells to bowel disease patients, and the field of isolation of colorectal epithelial stem cells.

## Claims

1. (A) A colorectal epithelial stem cell and/or a colorectal epithelial cell and (B) an extracellular matrix for use in the prevention or treatment of a human bowel disease:
wherein the cell is obtained by culturing a colorectal epithelial stem cell and/or a colorectal epithelial cell using a culture medium for *in vitro* culture of the colorectal epithelial stem cell and/or colorectal epithelial cell, and the extracellular matrix, the medium comprising serum albumin, Wnt3a, and r-spondin-1 as essential components and optionally further comprising epidermal growth factor (EGF), hepatocyte growth factor (HGF) and/or Noggin, and being serum-free;
wherein the culture medium is B-27 supplement free;
wherein the content of the serum albumin in the culture medium is in a range between 0.3% and 3% by weight;
wherein the extracellular matrix is collagen type I gel; and
wherein the cell is embedded in collagen type I gel and then cultured.

2. (A) The colorectal epithelial stem cell and/or a colorectal epithelial cell and (B) the extracellular matrix for the use of claim 1, in the treatment of a human bowel disease.

3. (A) The colorectal epithelial stem cell and/or a colorectal epithelial cell and (B) the extracellular matrix for the use of claim 2, wherein the method of the treatment comprises transplantation into a colon of a human.

## Patentansprüche

1. (A) Kolorektalepithelstammzelle und/oder Kolorektalepithelzelle und (B) extrazelluläre Matrix zur Verwendung zur Prävention oder Behandlung einer menschlichen Darmerkrankung:
wobei die Zelle durch Kultivieren einer Kolorektalepithelstammzelle und/oder einer Kolorektalepithelzelle unter Verwendung eines Kulturmediums für das In-vitro-Kultivieren der Kolorektalepithelstammzelle und/oder der Kolorektalepithelzelle und der extrazellulären Matrix erhalten wird, wobei das Medium Serumalbumin, Wnt3a und r-Spondin-1 als essentielle Bestandteile und gegebenenfalls außerdem epidermalen Wachstumsfaktor (EGF), Hepatozytenwachstumsfaktor (HGF) und/oder Noggin umfasst und serumfrei ist;
wobei das Kulturmedium frei von B-27-Ergänzung ist;
wobei der Serumalbumingehalt in dem Kulturmedium in einem Bereich zwischen 0,3 und 3 Gew.-% liegt;
wobei die extrazelluläre Matrix Kollagen-Typ-I-Gel ist und
wobei die Zelle in Kollagen-Typ-I-Gel eingebettet und dann kultiviert wird.

2. (A) Kolorektalepithelstammzelle und/oder Kolorektalepithelzelle und (B) extrazelluläre Matrix zur Verwendung nach Anspruch 1 zur Behandlung einer menschlichen Darmerkrankung.

3. (A) Kolorektalepithelstammzelle und/oder Kolorektalepithelzelle und (B) extrazelluläre Matrix zur Verwendung nach Anspruch 2, wobei das Behandlungsverfahren die Transplantation in einen menschlichen Dickdarm umfasst.

## Revendications

1. (A) Cellule souche épithéliale colorectale et/ou cellule épithéliale colorectale et (B) matrice extracellulaire à utiliser dans la prévention ou le traitement d'une infection intestinale humaine :
où la cellule est obtenue en cultivant une cellule souche épithéliale colorectale et/ou une cellule épithéliale colorectale en utilisant un milieu de culture pour une culture *in vitro* de la cellule souche épithéliale colorectale et/ou de la cellule épithéliale colorectale, et de la matrice extracellulaire, le milieu contenant de l'albumine sérique, Wnt3a et de la r-spondin-1 en tant que composants essentiels et comprenant en outre facultativement un facteur de croissance épidermique (EGF), un facteur de croissance hépatocytaire (HGF) et/ou de la Noggin, et étant exempt de sérum ;
dans lequel le milieu de culture est sans supplément de B-27 ;
dans lequel la teneur en albumine sérique dans le milieu de culture est comprise entre 0,3 % et 3 % en poids ;
dans lequel la matrice extracellulaire est un gel de collagène de type I ; et
dans lequel la cellule est incluse dans un gel de collagène de type I, puis cultivée.

2. (A) Cellule souche épithéliale colorectale et/ou cellule épithéliale colorectale et (B) matrice extracellulaire pour l'utilisation de la revendication 1, dans le traitement d'une infection intestinale humaine.

3. (A) Cellule souche épithéliale colorectale et/ou cellule épithéliale colorectale et (B) matrice extracellulaire pour l'utilisation de la revendication 2, où le procédé de traitement comprend une transplantation dans le côlon d'un être humain.
